(19) Europäisches Patentamt / European Patent Office / Office européen des brevets

(11) **EP 2 780 848 B1**

(12) **EUROPEAN PATENT SPECIFICATION**

(45) Date of publication and mention
of the grant of the patent:
**06.01.2021 Bulletin 2021/01**

(21) Application number: **12850199.6**

(22) Date of filing: **16.11.2012**

(51) Int Cl.:
*G16H 20/70* *(2018.01)*          *G16H 30/20* *(2018.01)*
*G06T 19/00* *(2011.01)*          *A61B 5/055* *(2006.01)*
*G09B 5/02* *(2006.01)*          *G09B 9/00* *(2006.01)*
*G16H 50/50* *(2018.01)*          *G09B 19/00* *(2006.01)*

(86) International application number:
**PCT/CA2012/001062**

(87) International publication number:
**WO 2013/071417 (23.05.2013 Gazette 2013/21)**

(54) **COMPUTER GENERATED THREE DIMENSIONAL VIRTUAL REALITY ENVIRONMENT FOR IMPROVING MEMORY**

RECHNERERZEUGTE DREIDIMENSIONALE VIRTUELLE REALITÄTSUMGEBUNG ZUR GEDÄCHTNISVERBESSERUNG

ENVIRONNEMENT DE RÉALITÉ VIRTUELLE TRIDIMENSIONNEL GÉNÉRÉ PAR ORDINATEUR, DESTINÉ À AMÉLIORER LA MÉMOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2011 US 201161560647 P**

(43) Date of publication of application:
**24.09.2014 Bulletin 2014/39**

(73) Proprietor: **Bohbot, Veronique, Deborah
Montreal, QC H2T2S5 (CA)**

(72) Inventor: **Bohbot, Veronique, Deborah
Montreal, QC H2T2S5 (CA)**

(74) Representative: **Rupprecht, Kay et al
Meissner Bolte Patentanwälte
Rechtsanwälte Partnerschaft mbB
Widenmayerstraße 47
80538 München (DE)**

(56) References cited:
**US-A1- 2005 216 243     US-A1- 2009 202 972**

- "Playstation - Wikipedia article", , 12 November 2011 (2011-11-12), XP055204961, Retrieved from the Internet: URL:https://en.wikipedia.org/w/index.php?title=PlayStation&oldid=460281800 [retrieved on 2015-07-28]
- "Functional magnetic resonance imaging - Wikipedia article", , 4 November 2011 (2011-11-04), XP055205133, Retrieved from the Internet: URL:https://en.wikipedia.org/w/index.php?title=Functional_magnetic_resonance_imaging&oldid=458943089 [retrieved on 2015-07-28]
- BROOKS ET AL.: 'The use of virtual reality in memory rehabilitation: Current findings and future diiections' NEUROREHABILITATION vol. 18, 2003, pages 147 - 157, XP008174056
- BOHBOT ET AL.: 'Grav matter differences correlate with spontaneous strategies in a human virtual navigation task' THE JOURNAL OF NEUROSCIENCE vol. 27, no. 38, 19 September 2007, pages 10078 - 10083, XP055154804
- BANNER ET AL.: 'The brain-derived neurotrophic factor Val66Met polymorphism is associated with reduced functional magnetic resonance imaging activity in the hippocampus and increased use of caudate nucleus-dependent strategies in a human virtual navigation task' EUROPEAN J. OF NEUROSCIENCE vol. 33, pages 968 - 977, XP055069300

## Description

### FIELD

The present disclosure relates generally to a computer generated three dimensional (3D) virtual reality (VR) environment for improving memory.

### BACKGROUND

In the prior art, advancements in computer technologies and high resolution graphic displays powered by graphics processing units (GPUs) have been used to create computer generated VR environments in which a user can navigate through virtual spaces - such as rooms, hallways, floors, buildings, streets, neighbourhoods, cities, landscapes, flight paths, etc. - by interacting with a navigational control. Often, the user is able to select a first person view such that the user may have a sense of being immersed in the virtual environment in which the user is navigating. This technology has been applied to various fields of endeavour, including computer games and vehicle simulators.

More recently, computer generated 3D VR environments have been used experimentally in new fields of endeavour, including experimental systems and methods for assisting users overcome their phobias. For example, VR systems have been developed to assist people with overcoming a fear of flying by having them participate in a controlled virtual flying environment. In another field of endeavour, 3D VR environments have been used to help patients reduce their experience of pain. For example, burn victims have been assisted by refocusing their attention away from the pain by having them engage in a 3D VR environment, such as a virtual snow world.

Yet other fields of endeavour are being explored in which 3D VR environments may be utilized to assist people. In particular, there is a need for a computer generated 3D virtual environment for assisting people with improving their memory. As background art, BOHBOT ET AL.: "Gray matter differences correlate with spontaneous strategies in a human virtual navigation task",THE JOURNAL OF NEUROSCIENCE, vol. 27, no. 38, 19 September 2007 (2007-09-19), pages 10078-10083 presents correlations on human virtual navigation tasks and grey matter changes.

### SUMMARY

The scope of the invention is defined by the claims. The following embodiments merely define examples, only those falling within the scope of the claims are part of the invention. The present disclosure is related to a computer generated 3D virtual environment for improving memory, and more particularly spatial, temporal, spatial-temporal, working and short-term memory.

In an aspect, provided is a computer-implemented system for generating a 3D virtual reality (VR) environment for improving memory (e.g. spatial, temporal, spatial-temporal, working and short-term memory), comprising: a control module configured to access at least one VR memory training module including one or more memory training tasks to be performed within a navigable three-dimensional (3D) environment; and a VR engine configured to execute the at least one VR memory training module with an output to a display, and an input from an interactive navigational controller. The system may further comprise means for performing one or more scans of brain structure and/or activity, whereby, the effectiveness of the at least one VR memory training module in targeting a selected region of the brain can be measured. In an embodiment, the control module is configured to determine which VR memory training module to retrieve and execute in dependence upon the measured effectiveness of a previous VR memory training module training session in targeting a selected region of the brain.

In another aspect, there is provided a computer-implemented method for generating a 3D virtual reality (VR) environment for improving memory (e.g. spatial, temporal, spatial-temporal, working and short-term memory). In an embodiment, the method comprises executing at least one VR memory training module including one or more memory training tasks to be performed within a navigable three-dimensional (3D) environment; displaying a navigable 3D environment via an output to a display; and receiving an input from an interactive navigational controller. The method may further comprise performing one or more scans of brain structure and/or activity, whereby, the effectiveness of the at least one VR memory training module in targeting a region of the brain can be measured. The determination of which VR memory training modules to retrieve and execute may be made based on the measured effectiveness of a previous VR memory training module training session in targeting a selected region of the brain.

In this respect, before explaining at least one embodiment of the system and method of the present disclosure in detail, it is to be understood that the present system and method is not limited in its application to the details of construction and to the arrangements of the components set forth in the following description or illustrated in the drawings. The present system and method is capable of other embodiments and of being practiced and carried out in various ways. Also, it is to be understood that the phraseology and terminology employed herein are for the purpose of description and should not be regarded as limiting.

## BRIEF DESCRIPTION OF THE DRAWINGS

FIG. 1 shows a schematic block diagram of a system in accordance with an embodiment.

FIG. 2 shows a schematic flow chart of a method in accordance with an embodiment.

FIGS. 3A to 3D shows various fMRI scans of activity (FIGS. 3A and 3B) and grey matter (FIGS. 3C and 3D) in the brain resulting from performing memory testing that dissociates between spatial and response navigational strategies in accordance with various embodiments.

FIG. 4 shows a graph of correlation between the Montreal Cognitive Assessment (MoCA) test which is sensitive to dementia and a learning test in a group of healthy older adults.

FIG. 5 shows experience-dependent growth in the hippocampus and striatum of mice trained in different versions of a maze.

FIGS. 6A and 6B show an illustrative randomization procedure using a stratified randomization method.

FIG. 7 shows an illustrative list of different VR memory training modules in accordance with an embodiment.

FIGS. 8A and 8B show illustrative screenshots taken from a discrimination tasks module.

FIG. 9 shows an illustrative top view of rooms and a list of objects to find as specified by the discrimination tasks.

FIGS. 10A and 10B show illustrative screen shots taken from VR memory training modules for discrimination and spatial memory tasks.

FIG. 11 shows an illustrative top view of an answer key having a list of instructions showing the relationship between the locations of various rooms that participants learn for participants to follow.

FIGS. 12A and 12B show illustrative screenshots of a VR memory training module for object location tasks.

FIG. 13 shows an illustrative example of a top view of a virtual environment with objects for use with the VR memory training module of FIGS. 12A and 12B.

FIGS. 14A and 14B show illustrative screenshots of a VR memory training module for Spatio-Temporal Order tasks.

FIGS. 15A - 15J show illustrative screen shots of a VR memory training module for placing landmarks in temporal order in the Spatio-Temporal Order tasks.

FIGS. 16A and 16B show illustrative screenshots taken from VR memory training modules for navigation tasks.

FIG. 17 shown is a top view map of a small city with landmarks listed from a VR memory training module for navigation tasks of FIGS. 16A and 16B.

FIG. 18 shows a VR memory training module for a number-letter sequencing task in accordance with an embodiment.

FIG. 19 shows a VR memory training module for an N-back task in accordance with an embodiment.

FIG. 20 shows a VR memory training module for a counting forwards and backwards module in accordance with an embodiment.

FIG. 21 shows a VR memory training module for another counting forwards and backwards module in accordance with another embodiment.

FIG. 22 shows a VR memory training module for memorizing numbers of coloured cards encountered along a path in accordance with an embodiment.

FIG. 23 shows an experimental design in accordance with an embodiment.

FIG. 24 shows an illustrative series of transfer tests administered before and after the memory training in accordance with an embodiment.

FIG. 25 shows another illustrative series of transfer tests administered before and after the memory training in accordance with another embodiment.

FIGS. 26A and 26B show two illustrative transfer tests in accordance with yet another embodiment.

FIG. 27 shows a 4-on-8 virtual maze transfer test in accordance with an embodiment.

FIGS. 28A - 28C show various screen shots of a wayfinding transfer test in accordance with an embodiment.

FIGS. 29A and 29B show a design of a go/no-go transfer test in accordance with an embodiment.

FIG. 30 shows a design of a concurrent spatial discrimination learning task transfer test in accordance with an embodiment.

FIG. 31 shows illustrative percentage improvement calculations in accordance with an embodiment.

FIGS. 32A and 32B show graphic results of pre- and post-memory training for performing various tasks in healthy older adults.

FIGS. 33A and 33B show graphic results of pre- and post-memory training for performing various other tasks in healthy older adults.

FIGS. 34A show fMRI scans of increased fMRI activity in the HPC of healthy older adults who underwent memory training in accordance with an embodiment and FIG. 34B show no such increased fMRI activity in the controls.

FIGS. 35A show structural MRI scans of induced growth in the HPC (at the cross hair) of healthy older adults who underwent memory training in accordance with an embodiment as measured by VBM. In addition to growth in the HPC, the memory training induced growth in areas of the brain throughout the neocortex, including the entorhinal cortex, perirhinal cortex, parahippocampal cortex, orbito-frontal cortex, occipital cortex, parietal cortex, temporal cortex and other regions of the frontal cortex and amygdala. FIG. 35B show no such increased structural MRI growth in the controls.

FIGS. 36A - 36C show bar graphs of the performance of Mild Cognitive Impairment patients during VR memory training in accordance with an embodiment.

FIGS. 37A - 37B show additional bar graphs of the performance of Mild Cognitive Impairment patients during VR memory training in accordance with an embodiment.

FIGS. 38A - 38C show comparative bar graphs of pre- and post- VR memory training performance in Mild Cognitive Impairment patients in accordance with an embodiment.

FIGS. 39A - 39B show comparative bar graphs for a group of Mild Cognitive Impairment patients on the memory training in accordance with an embodiment.

FIGS. 40A - 40C show bar graphs of the performance of a group of Mild Cognitive Impairment patients on the memory training in accordance with an embodiment.

FIG. 41 shows an illustrative example of a placebo control that may be used.

FIG. 42 shows illustrative examples of pre- memory training structural MRI scans of four patients with Mild Cognitive Impairment.

FIGS. 43A and 43B show pre-memory training functional MRI scans of Mild Cognitive Impairment patient and Healthy older adult participants, average of first experimental trial. The figures show no fMRI activity in the hippocampus prior to the memory training.

FIG. 44 shows post-memory training functional MRI minus pre-memory training functional MRI scans of two patients with Mild Cognitive Impairment. This figure shows recovery of fMRI activity in the hippocampus of patients with dysfunction to the hippocampus such as Mild Cognitive Impairment patients.

FIG. 45 shows a generic computer device which may provide a suitable operating environment for various embodiments.

## DETAILED DESCRIPTION

As noted above, the present disclosure relates to a computer generated 3D virtual environment for improving memory. While the present system and method may be used to train different types of memory, including spatial, temporal, spatial-temporal, working and short-term memory, the discussion below focuses on training spatial memory as illustrative examples of various embodiments.

Also, while examples of the items to be remembered include objects, letters and digits, this is illustrative and not meant to be limiting. For example, other things to be remembered may include faces, animals, words, sentences, stories, rooms, or landmarks, for example. Again, this list of things to remember is not meant to be limiting. In addition, any sensory stimuli could be used, including auditory, visual, olfactory, somato-sensory, motor, etc.

In the description below, references to discrimination tasks may involve perceptual discrimination rather than involving memory. However, it will be appreciated that spatial memory improvement includes various components important for spatial memory, such as perception, temporal, spatio-temporal, working and short-term memory. This list is not meant to be restricted to a particular definition or semantic description of memory. For example the types of memory described above include different definitions of memory such as relational memory, episodic memory, semantic memory, declarative memory, temporary memory. This is not an exhaustive list of the types of memory but only examples to convey the breadth of the definition of memory.

For the purposes of the present disclosure, the list of acronyms below have the following meaning.

List of Acronyms

| | |
|---|---|
| 4/8VM: | 4-on-8 Virtual Maze |
| AD: | Alzheimer's disease |
| BIS-11: | Barratt Impulsiveness Scale 11 |
| BOLD: | Blood Oxygenation Level Dependent |
| CSDLT: | Concurrent Spatial Discrimination Learning Task |
| CN: | Caudate Nucleus |
| CT: | Computerized Tomography |
| DST: | Digit Symbol Test |
| ET-CT: | Experimental Trials - Control Trials |
| FSAQ: | Functional Spatial Abilities Questionnaire |
| FWHM: | full-width at half-maximum |
| GDS: | Geriatric Depression Scale |
| HPC: | Hippocampus |
| INSECT: | Intensity Normalized Stereotaxic Environment for the Classification of Tissues |
| MCI: | Mild Cognitive Impairment |
| MMSE: | Mini-Mental State Examination |
| MoCA: | Montreal Cognitive Assessment |
| MRI: | Magnetic Resonance Imaging |
| NLS: | Number-Letter Sequencing |
| PC: | Placebo Control |
| PSS: | Perceived Stress Scale |
| QOL: | Quality of Life |
| RAVLT: | Rey Auditory Verbal Learning Task |
| ROI : | Region of interest |

ROCF: Rey-Osterrieth Complex Figure
SEQ: Self-Esteem Questionnaire
SMIP: Spatial Memory Improvement Program
S-R: Stimulus-Response
TONI-III: Test of Nonverbal Intelligence III
VBM: Voxel-Based Morphometry
WAIS-R: Wechsler Adult Intelligence Scale
WM: Working Memory

Regions of the Brain Associated with Memory

Controlled studies have shown that in order to find our way and move adaptively within a new environment, humans often spontaneously adopt different navigational strategies which rely on different parts of the brain. For example, to reach a target location, a person may use a "spatial memory strategy" by learning the relationships between environmental landmarks (i.e. stimulus-stimulus associations). This strategy is a form of explicit memory based on a cognitive map which allows a target to be reached in a direct path from any given direction. This type of flexible navigation has been shown to depend upon the hippocampus (HPC) region of the brain. Alternatively, one can navigate without knowledge of the relationships between environmental landmarks, but instead, by using a series of turns at precise decision points or stimuli (e.g. turn left at the corner, then turn right after the park etc.). The successful repetition of this latter non-spatial strategy leads to a "response strategy" (stimulus-response associations) known to involve the caudate nucleus (CN) region of the brain, a form of implicit memory, automatization of behavior or habit. The frontal cortex, another region of the brain, which is involved in short-term memory or working memory (i.e. holding onto multiple pieces information for a limited time in order to make this information available for further information-processing), planning, decision-making and inhibition was shown to be involved in modulating which strategy is used at a given time. The amygdala, a region of the brain involved in emotions, stress and fear has been shown to promote response strategies.

In a previous study, 50 young healthy participants performed a virtual navigation task (a "virtual maze task") on a computer monitor which could be solved by using either of these two strategies - i.e. the "spatial memory strategy" or the "response strategy". The participants had to learn the locations of objects hidden at the end of paths extending from a radial maze. A probe trial that involved the removal of all landmarks was used to identify the participants adopting a spatial strategy because it was predicted that only this group would show an increase in errors. Based on self report and the probe trial, it was found that about half of the participants spontaneously used the response strategy. They made fewer errors on the probe trial and reported following a pattern of open and closed arms or a series of directions (e.g. take the second path to the left, then take the next left) from a given starting point or stimulus. The other half of the participants spontaneously used spatial memory (i.e. the reported using two landmarks and did not use a pattern of opened and closed arms). The group using spatial memory made significantly more errors on the probe trial and reported learning the locations of target objects in relation to multiple landmarks. In this experiment, it was found that the response strategy was more efficient than the spatial memory strategy, as evidenced by fewer errors and less time to complete the task. With further training, 40% of the people using the spatial memory strategy shifted to the more efficient response strategy, as has been demonstrated in rats in earlier studies.

A functional Magnetic Resonance Imaging (fMRI) study conducted during the memory testing was done showed that the HPC region of the brain was significantly activated only in spatial learners, whereas the CN region showed significant sustained activity in response learners. Response learners showed no activity in the HPC. Voxel Based Morphometry (VBM) has been used to identify brain regions co-varying with the navigational strategies used by individuals. Results showed that spatial learners had significantly more grey matter in the HPC and less grey matter in the CN as compared to response learners. On the other hand, response learners had more grey matter in the CN and less grey matter in the HPC. Further, the grey matter in the HPC was negatively correlated to the grey matter in the CN, suggesting a competitive interaction between these two brain areas. In a second analysis, the grey matter of regions known to be anatomically connected to the HPC, such as the amygdala, parahippocampal, perirhinal, entorhinal and orbito-frontal cortices were shown to co-vary with grey matter in the HPC. In other words, spatial learners had more grey matter in the HPC but they also had more grey matter in the network of anatomically connected areas described above which included the amygdala and cortex. Since low grey matter in the HPC is a risk factor for Alzheimer's disease as well as cognitive deficits in normal aging and other neurological and psychiatric disorders that affect the HPC such as Depression, Bipolar disorders, Schizophrenia, Post-Traumatic Stress Disorders, Diabetes, Addiction, Dementia, Parkinson's disease (with dementia) or any other disorder affecting memory and the HPC, these results have important implications for cognitive training programs that aim at functional recovery in these brain areas.

Memory Impairment

Through many years of research in this field, the inventor has come to appreciate and recognize a need for developing an effective system and method for improving memory to help protect against or to slow the degeneration of the HPC and other regions of the brain that occurs with normal aging, and with various cognitive impairments and diseases.

Recent studies have shown that the percentage proportion of the Canadian population over age 65 will climb from 11.6% in 1991 to 16% in 2016, and 23% in 2041. By the year 2050, 16% of the world population will be over the age of 65. In the US, 20% of the population is expected to over the age 65 in 2050 and in Japan, 38% of the population is expected to be over 65 in 2050. The Canadian Study of Health and Aging has documented a current prevalence of 8% for dementia in this group of citizens over the age of 65, and the prevalence rises exponentially with age. There is currently an incidence of 60,000 new cases of dementia each year in Canada. Alzheimer's disease (AD) is the most common form of dementia, accounting for at least 65% of cases, or about 200,000 people in Canada in 1998. It is severely disabling and a major human, social, and economic burden. All of this makes prevention of AD a major public health issue in Canada, and in many other jurisdictions with a growing percentage of elderly people in their populations.

Mild cognitive impairment (MCI) is an intermediate cognitive state between normal aging and AD. Patients with MCI suffer subjective memory impairments while being functionally autonomous. Approximately 44% of MCI patients recover. However, patients with amnestic MCI have memory impairments and AD pathology. The first regions of the brain to show AD pathology are the entorhinal cortex, the HPC, and with disease progression, the neocortex which includes the Frontal cortex. Reductions in HPC volumes were found to be good predictors of ensuing AD.

Interestingly, the HPC is a structure which has been shown to have neurogenesis across the entire life span in rodents and in primates. This neurogenesis in the HPC is stimulated by learning and memory paradigms that were shown to increase cellular survival in adult primates. As such, learning and memory paradigms may help cellular survival and synapse development in the HPC of MCI patients by focusing on the very region in which the pathology emerges.

A number of memory intervention studies have proven successful in helping alleviate memory impairments in MCI patients. However, most of these studies have assumed involvement of the HPC but have not actually tested their hypothesis with brain imaging or by testing patients with lesions specific to the HPC. Memory intervention studies that target the HPC may be most effective in alleviating symptoms of MCI.

As will be explained in further detail below, the use of VR has enabled the production of innovative learning and memory paradigms proven to be sensitive to training various regions of the brain. Based on these findings, the inventor has developed a spatial memory improvement program (SMIP) with a plurality of training programs designed to stimulate the HPC and cortex. Initial results in healthy older adults (59-75 years of age) showed that the SMIP improved memory, increased activity in the HPC, and induced growth in the HPC and cortex, as evidenced by functional and structural Magnetic Resonance Imaging. Further, participants found the SMIP to be enjoyable and its similarity to real life environments allowed a direct transfer to their everyday lives. Participants testified to being more autonomous and confident, which shows that the SMIP was helpful in improving their quality of life. Thus, the inventor believes that SMIP is a promising tool for promoting healthy aging and reducing the symptoms associated with MCI.

System and Method for Improving Memory

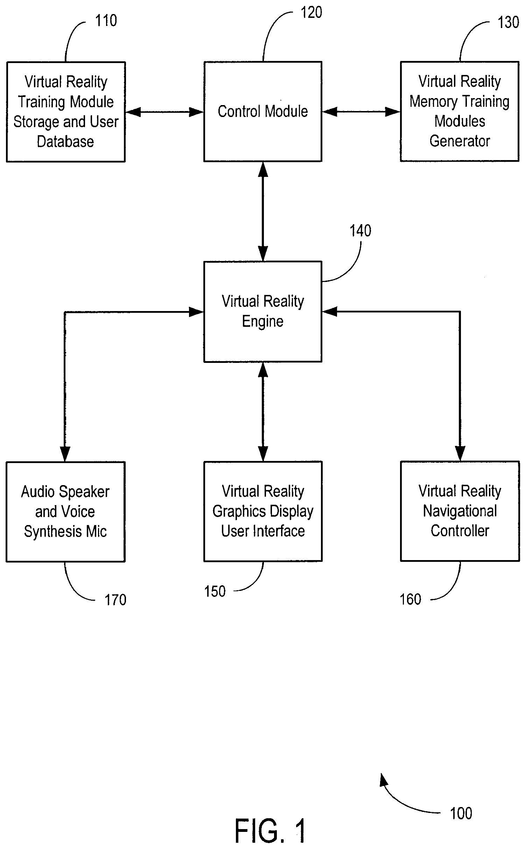

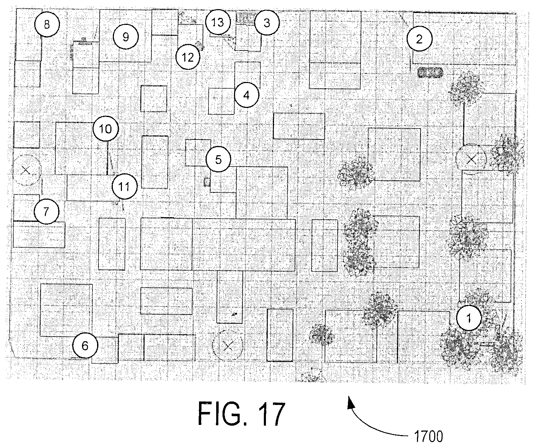

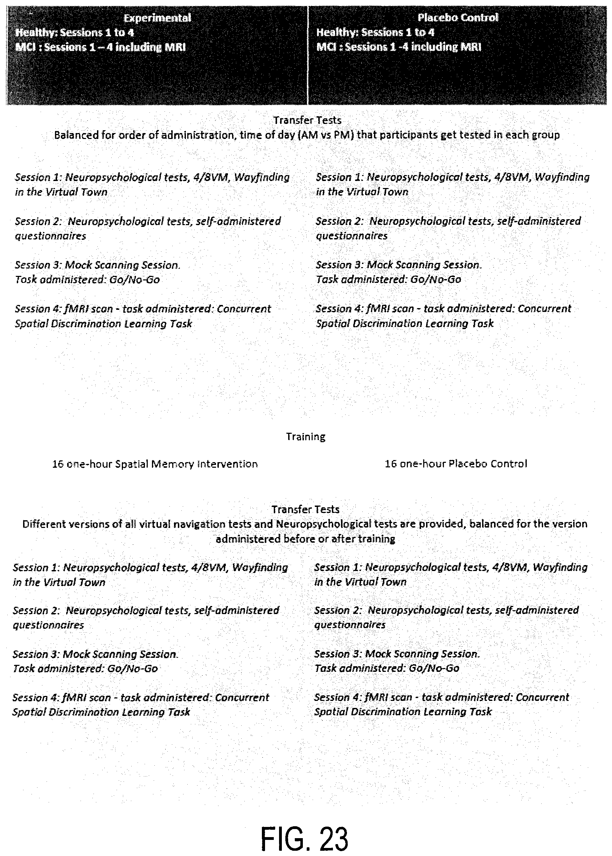

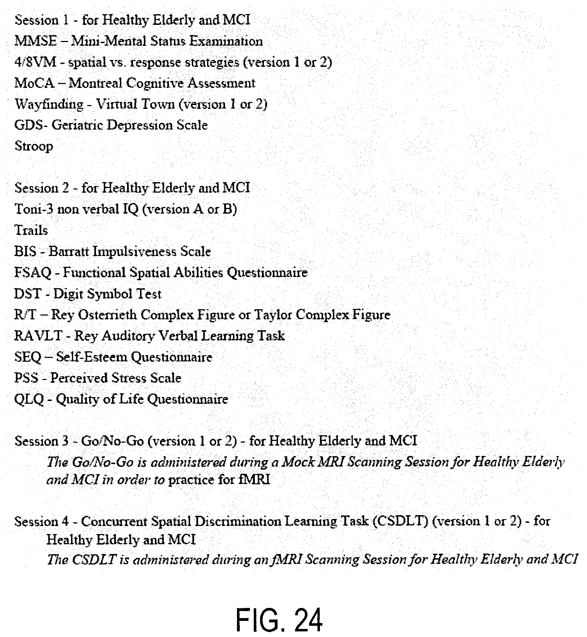

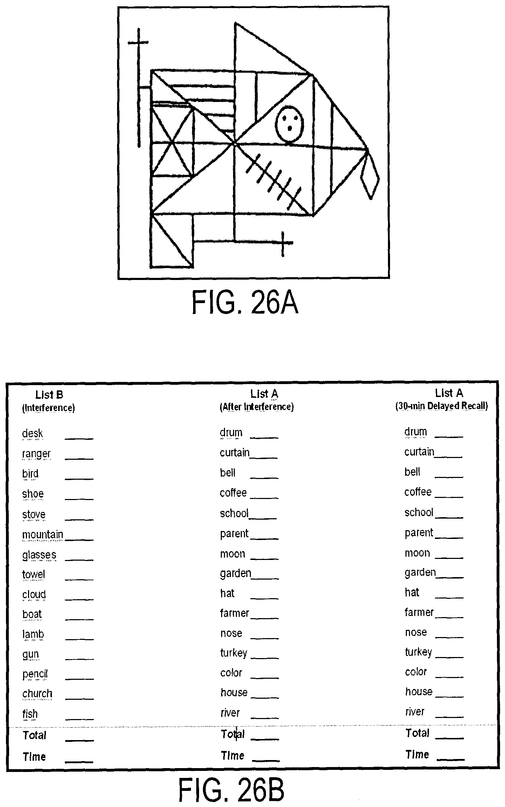

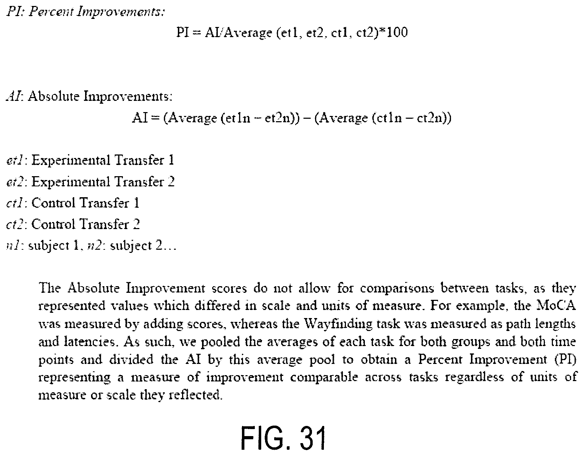

As will now be described in detail with reference to the accompanying FIGS. 1 to 41, the inventor has developed a computer generated 3D virtual environment for executing a plurality of memory training programs which are designed to improve memory and navigational skills in various populations including MCI and healthy participants relative to placebo control (PC) groups, and also improve memory and increase blood flow and grey matter in the HPC relative to controls. By exercising and developing the HPC region and other regions of their brains, participants are able to show a measurable improvement in their memory function.

Now referring to FIG. 1, shown is a schematic block diagram of a system 100 in accordance with an illustrative embodiment, which is not meant to be limiting. As shown, system 100 includes a virtual reality (VR) memory training module storage and user database 110 operatively connected to a control module 120. Control module 120 is adapted to operatively connect to the database 110 in order to access the various training modules and data about various users or participants. Control module 120 is also operatively connected to a VR memory training module generator 130 for generating additional training modules, as will be described in more detail further below. Control module 120 is further operatively connected to a VR engine 140 for creating a VR environment as discussed in more detail below. VR engine 140 is in turn operatively connected to a VR graphics display user interface 150, a VR navigational controller, and an audio speaker / voice synthesis microphone 160. These various modules will now be described in more detail.

In an embodiment, control module 120 may be hosted on a generic computing device with an operating system for running various software modules and the memory training modules as described herein. As noted, control module 120 is adapted to access one or more suitable memory training modules stored in database 110. The selection of which memory training module is retrieved for execution may be determined by the control module based on a particular user's profile as also stored in database 110. (It will be appreciated that the user profiles may also be stored in a separate database on another hardware device, and the storage location of the user profiles and the memory training modules are not meant to be limiting.)

In an embodiment, control module 120 is adapted to keep track of a user's progress through a memory training program. Based on a user's initial profile, and feedback obtained during the course of a memory training program, control module 120 may determine which memory training modules to retrieve and execute. As training may be scheduled over a number of weeks, months, or even years, control module 120 is configured to keep track of the progress of training for each and every user or participant. Control module 120 is also configured to keep track of which memory training modules have been used for a particular user, and how many times a particular memory training module has been used for the particular user.

In another embodiment, control module 120 may determine if a particular memory training module has been offered to and executed by a particular user more than a pre-determined number of times. For example, control module 120 may be configured to limit the number of repetitions of any particular module to between 3 and 5 repetitions. By limiting the number of times a particular memory training module is repeated, control module 120 prevents the participant from simply relying on a response strategy, or implicit memory developed from habit.

In an embodiment, control module 120 is operatively connected to a VR engine 140 for generating a navigable, VR environment for the memory training modules. For example, the VR engine 140 may be configured to generate a virtual 3D environment on a VR graphics display user interface 150 to interact with a user. The user interface 150 may be, for example, a computer display suitable for generating a graphics output at a sufficiently high frame refresh rate to provide a user with a sense of motion through a virtual 3D environment. A VR navigation controller 160 may be, for example, a mouse, joystick, trackball, response box, or direction keys on a keyboard for navigating within the VR environment.

In an embodiment, the user interface 150 may be a type of graphics display provided on a large screen (e.g. 3 meter wide screen displaying 2D or 3D depth perceptual stimuli), in a totally black room, which produces a 3D environment. It can also be displayed on a regular computer screen or on any type of computerised display (e.g. game station, Wii®, iPad®, iPhone®, Android®). Alternatively, it can be displayed on VR glasses or goggles (not shown) that may be worn around the eyes of a user or participant. In this embodiment, as the user is wearing the graphics display and peripheral vision may be partially or fully blocked, the user may feel much more immersed in the VR environment. If the VR glasses or goggles are fitted with accelerometers to detect motion and orientation, the user may control the direction of view of the VR environment by simply moving his head to the direction he would like to see. This may be supplemented by a navigation sensor worn on the hands or legs, or by a body position detector such as the Microsoft Kinect® system to initiate movement towards a particular direction.

In another embodiment, VR engine 140 is also operatively connected to an audio speaker / voice synthesis microphone 170 to facilitate audio interaction with the VR engine 140 and control module 120. For example, speaker / mic 170 may be used to provide instructions to the user during the course of a memory training program, and may also be used to receive responses, questions or commands from the user.

By providing a 3D VR environment, which in addition to a visual user interface may also include motion feedback and audio interaction, the participant may be more fully engaged with each memory training program. Further, the HPC is a multimodal association area that receives auditory, olfactory, somatosensory as well as visual information. As such, multi-modal stimulation within the domain of spatial memory fully engages the HPC.

In another embodiment, a VR helmet may be provided which may include sensors for conducting measurements of brain activity, and which may also include sensors for identifying which regions of the brain are the most active. Such sensors may be used to measure pre-training brain activity, post-training brain activity, or brain activity during the course of conducting a VR memory training session.

In an embodiment, control module 120 may be configured to adapt a memory training program in dependence upon feedback obtained from each user participating in a memory training program. For example, control module 120 may determine how long it takes a particular user to complete a given memory training module, and how many tasks in each training module are successfully completed without errors. Control module 120 may also receive feedback from sensors indicating the level of brain activity in particular regions of the brain. Based on this feedback, control module 120 may modify the training program to either increase or decrease the level of difficulty of the selected memory training modules. The level of difficulty may be increased, for example by increasing the number of tasks, placing a larger number of objects in a VR environment for recall, or making the VR environment more complex with the addition of doors, hallways, and paths and reduction of landmarks. Similarly, the level of difficulty can be decreased by reducing the number of tasks, using fewer objects, or making the VR environment less complex with more landmarks, and a reduced number of doors or paths for selection.

In addition to direct measurement of user results in completing a memory training module, control module 120 may obtain additional feedback by directly engaging the user to answer questions following completion of a memory training module. For example, control module 120 may ask the user to rate the perceived level of difficulty of a particular training module, and may adapt the training program based on the user's direct feedback.

In another embodiment, control module 120 may be configured to receive physiological feedback, e.g. but not limited to heart rate, heart coherence, Electro Encephalogram (EEG), EEG coherence, measures of activity levels by body motion detection, or an MRI scan of a participant's brain structure and/or activity during, or shortly after completing a memory training module. As will be described in more detail below, areas of the brain which have been stimulated and activated by the memory training module may be identified by highlighting the degree of increased activity in a particular region of a brain measured with MRI in terms of grey matter and blood flow. For an example an MRI scan could be used to determine the duration and frequency of the training module.

In an embodiment, control module 120 may generate new training modules for use in a participant's memory training program based on feedback received from a user during the course of her participation in the memory training program. For example, the new training modules may be based on a VR environment containing a standard set of tasks, objects, number of paths, etc. which need to be modified to either increase or decrease the level of difficulty. Such customized memory training modules may then be stored in database 110 in order to be offered to a particular user based on their individual profile. Based on measurement of any improvements in results, control module 120 will determine if the customized new training modules have been more effective or less effective. Over the course of time, based on measured feedback, control module 120 may determine to what degree to either increase or decrease the level of difficulty to try to optimize the memory training program. However, an override of the control module 120 may be initiated if necessary.

In another embodiment, control module 120 may include a virtual coach for providing feedback and coaching to a participant interacting with a memory training module. In an embodiment, the virtual coach may be represented as an avatar within the VR environment with which the user can interact. For example, the virtual coach may appear at the start of each memory training module to provide verbal and/or text guidance on how the user should perform the memory training tasks in the module. In this manner, the present system and method may achieve better training outcomes by ensuring that the user performs the memory training tasks as intended.

Similarly, in the course of a memory training session, if the user should appear to be having difficulty, the virtual coach can appear to provide clues and encouragement for the user to continue the memory training session. Upon completion of a memory training session, the virtual coach can provide the user with feedback on how the user did, and may provide the user with congratulations for doing well, or providing encouragement and providing advice on how the user can improve further. It will be appreciated that the virtual coach's avatar may take any form, including a digital representation or photo of a person known to the user, such that the user feels more comfortable with interacting with the system. The avatar can be standard or it can be of the user's choice, including a custom made avatar of different ethnicity, culture, language, age, sex, and physical appearance (in terms of physical body features and clothing).

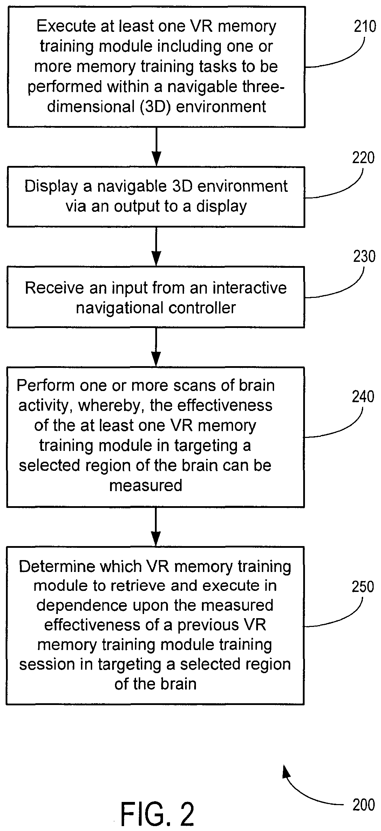

Now referring to FIG. 2, shown is a schematic flow chart of a method 200 in accordance with an embodiment. As shown, method 200 begins and at block 210 method 200 executes at least one VR memory training module including one or more memory training tasks to be performed within a navigable three-dimensional (3D) environment. Method 200 then proceeds to block 220, where method 200 displays a navigable 3D environment via an output to a display. Method 200 then proceeds to block 230, where method 200 receives an input from an interactive navigational controller.

In an embodiment, method 200 further proceeds to block 240, where method 200 performs one or more scans (e.g. a pre-training scan, a post-training scan, or in-training scan) of brain structure and/or activity, whereby, the effectiveness of the at least one VR memory training module in targeting a selected region of the brain can be measured. Method 200 then proceeds to block 250, where method 200 further determines which VR memory training module to retrieve and execute in dependence upon the measured effectiveness of a previous VR memory training module training session in targeting a selected region of the brain.

The remainder of the specification will provide a detailed discussion of an illustrative embodiment of the present system and method.

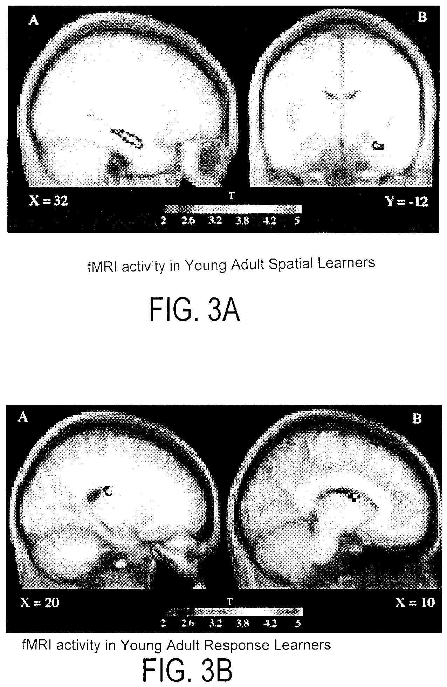

In an illustrative embodiment, a 4-on-8 virtual maze (4/8VM) virtual navigation task was established to serve to distinguish between different learning strategies. In the first part of the task, participants had to retrieve four objects at the end of four open paths out of eight that extend from a central platform. In the second part, the objects were placed in the paths that were previously blocked and participants were asked to retrieve them. Spatial learners were distinguished from response learners using probe trials in which environmental landmarks were removed. As shown in FIGS. 3A and 3B, an fMRI task confirmed that the participants who employed spatial strategies, but not those who used response strategies, showed increased activity in the HPC relative to baseline. More particularly, FIGS. 3A and 3B show regions of activity in the hippocampus (HPC), and caudate nucleus (CN) found in the spatial learning group and response learning group respectively. The t-maps are superimposed onto the anatomical average of all participants and displayed in the sagittal and coronal plane. In FIG. 3A, the activity shown is in the right HPC when contrasting the experimental and control conditions of the spatial learning group minus those of the response learning group in the first scan ($x = 32$, $y = -12$, $z = -22$, $t = 4.17$). In FIG. 3B, the activity shown is in the right CN found in the response learning group (scan 5) ($x$

= 14, y = -8, z = 22; t = 4.04). The response group, on the other hand, showed sustained increased activity in the CN.

The hypothesis that spatial strategies on the 4/8VM are associated with the HPC was further supported in a lesion study. Patients were tested after undergoing a unilateral surgical resection of the medial temporal lobe, which includes the HPC, for the treatment of epilepsy. In line with earlier fMRI results, spatial learners with damage to the HPC were significantly impaired on the 4/8VM relative to response learners with similar damage. Thus, response strategies involve a neural circuitry that is independent of the HPC whereas spatial strategies critically require the HPC.

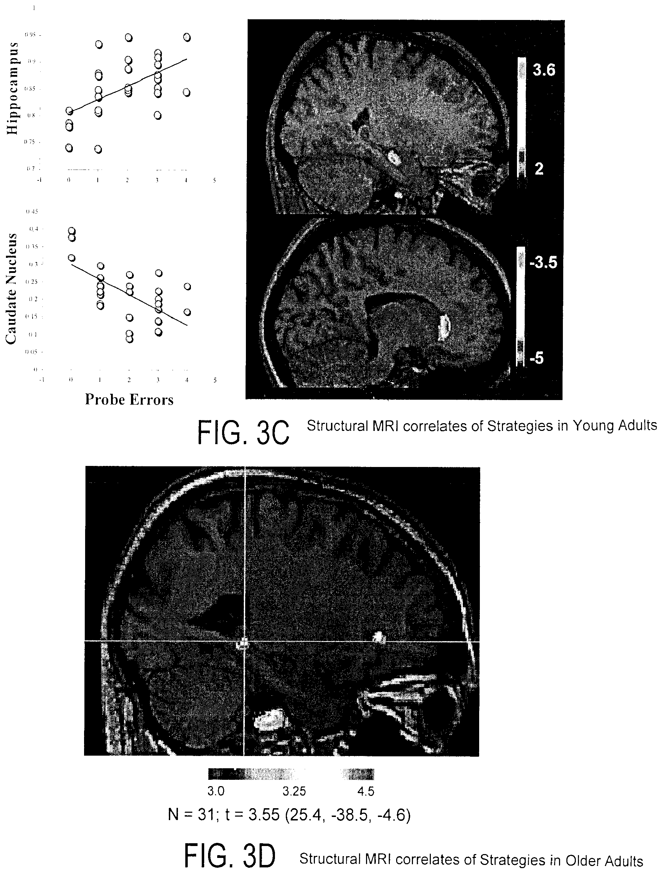

Neuroanatomically, spatial learners have more grey matter in the HPC than response learners. In another study, thirty anatomical MRI scans were obtained from young adult participants (average age: 27.9). Voxel Based Morphometry (VBM), a completely automated analysis, revealed that the number of errors on the probe trial, in which all spatial landmarks are removed, significantly correlated with grey matter density in the right HPC. More particularly, FIG. 3C shows the regression analyses between the grey matter density (HPC and CN) and the errors made by the entire group of young adult human participants while performing the probe trial. The right side of FIG. 3C shows the results superimposed onto an anatomical MRI and displayed in the sagittal plane. Grey matter density in the right HPC at the peak (x = 24, y = -13, z = -20; t-statistic = 3.55) was correlated with spatial learning strategies (r = 0.56, p < 0.005; top panel) whereas the density in the head of the CN at the peak (x = -14, y = 28, z = 4; t-statistic = -4.33) was correlated with response learning strategies (r = -0.63, p < 0.005; bottom panel). Aside from a negative correlation between probe errors and the tail of the CN (x = -26, y = -32, z = 3; t-value = -4.07; with a correlation coefficient r = 0.56, p < 0.005), no other region of the brain crossed the threshold for significance corrected for multiple comparisons. The vertical bars illustrate the range of t-statistical values.

Interestingly, the response group had the lowest grey matter density in the HPC and highest in the CN. These findings are consistent with the study of London taxi drivers which showed a positive correlation between the volume of the posterior HPC and experience driving a taxi. The present system and method is this study is the first to associate HPC to navigation in healthy young adults without a particular expertise.

Now referring to FIG. 3D, shown is a regression analysis of grey matter density of structural MRIs and scores on probe trials of a concurrent spatial discrimination learning task in healthy older adults. Results show that grey matter density in the right HPC at the peak (x = 25.4, y = - 38.5, z = -4.6; t-statistic = 3.55) significantly correlated with higher scores on the probe trials of the Concurrent Spatial Discrimination Learning Task, a task which requires the use of a spatial memory strategy. Other areas of the brain that correlated with spatial memory include the fusiform gyrus and frontal cortex, however, these peaks do not cross the statistical threshold after the Bonferonni correction for the entire volume.

A study has shown that a greater proportion of human older adults use response strategies suggesting changes across the life span. It was found that 85% of children (N=243, mean age: 8.0) used spontaneous spatial memory strategies as opposed to 47.4% in young adults (N=175, mean age: 25.1), and 39.3% in older adults (n=112, mean age: 66.4) ($X^2$ = 64.49, p < 0.0001). Similar results were found in MCI patients. Out of three MCI patients tested, two spontaneously used a response strategy and one used a spatial strategy. Although the sample size is low, the proportion of spatial and response strategies is similar to that in the healthy older adult population. Research performed on young adults showed no relationship between previous gaming experience and spatial memory performance, suggesting that video game experience is unlikely to explain changes across the life span. In sum, the data suggest that in contrast to children, there is evidence for increasing use of response strategies across the life span. This is consistent with a memory study in which PET imaging revealed age-related changes towards using the CN in older adults relative to the HPC in young adults.

The use of response strategies in healthy older adults may be associated with a greater risk of dementia. Low HPC grey matter was shown to be a predictor of the conversion of MCI to AD. Since spatial strategies are associated with increased HPC grey matter, they may also be associated with reduced risks of AD. Results in the inventor's laboratory support this hypothesis:

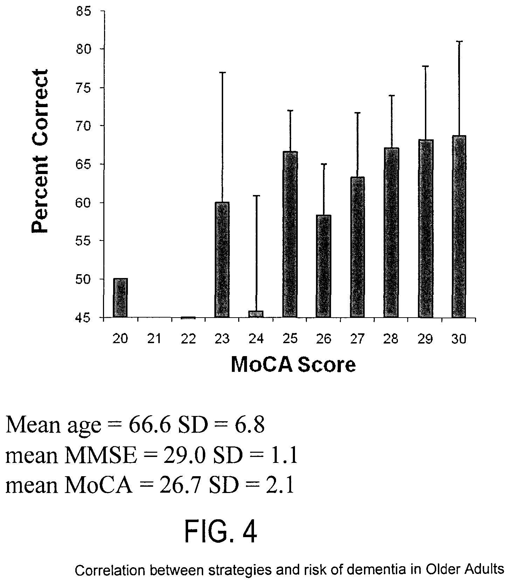

FIG. 4 graphically illustrates a correlation analysis in 85 older adults (mean age = 66.6 yrs) which shows a negative correlation between MoCA and spatial memory strategies (R2=0.0439, p<0.05). This suggests that older adults employing spatial strategies have better overall cognition and that participants employing responses strategies have the poorest scores on the MoCA test, indicative of a greater risk of dementia.

The spatial memory correlation with HPC grey matter reported with VBM in human adults was replicated in a collaborative mouse imaging study with the inventor's laboratory, in which spatial memory training in adult mice induced growth in the CA fields of the HPC whereas stimulus-response training did not.

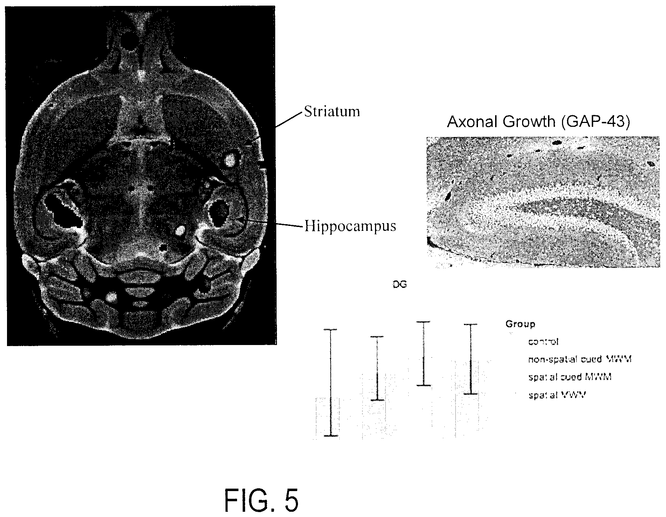

Shown in FIG. 5 is a postmortem VBM contrast between seven Tesla MRI scans of mice trained in the spatial version of a Morris Water Maze (MWM) task against the MRI scans of mice trained in the response version (response-cued MWM). One group of mice received the standard spatial memory training of the Morris Water Maze task, in which external visual landmarks around the maze could be used to find a hidden escape platform. Another group received response training, in which the escape platform was indicated by a flag and the external landmarks were hidden behind a curtain. After five days of training, the mice were euthanized, injected with a contrast agent, and scanned on a 7 Tesla MRI scanner. Growth in the HPC of spatial learners is labeled in blue. Growth in the Striatum of response learners is identified in red. Results showed a significant increase in HPC grey matter in the spatial group (12% in dentate gyrus of the HPC and 16% in CA1 layer of HPC) and a significant increase in striatal grey matter in the response group (11% in CN and putamen). Note that the CN and Putamen, which form the Striatum in humans, are merged into one structure called the striatum in rodents. Cytoarchitectural analyses showed no difference in cell body counts. However, the lower figure shows a significant increase in GAP-43 labeling in the dentate gyrus of the HPC in the mice trained on the spatial versions relative to control and response-cued MWM-trained mice. GAP-43 is present in pre-synaptic terminals and is evidence for axonal growth. The mouse study suggests that spatial learning promotes growth of grey matter in the HPC and that response learning does not.

In other words, certain types of learning, such as response learning, do not impact HPC grey matter. The causal link between spatial memory training and growth in HPC grey matter shown here and previously inferred in a human VBM study provides supportive evidence for a spatial memory-based intervention program.

The above lines of evidence point to the necessity of dissociating spatial and response learning strategies in order to specifically target the HPC in a cognitive improvement program. Earlier studies suggest that over 60% of healthy elderly and MCI patients will spontaneously use response strategies. As such, cognitive intervention programs based on memory training that do not dissociate strategies may or may not engage the HPC. Should other regions of the brain, such as the CN, be engaged, the intervention method could have far less of an impact on improving AD outcomes. Thus, in an embodiment, the proposed intervention the SMIP may be based on tasks that have been shown to be sensitive to the function of a specific region of the brain, such as the HPC.

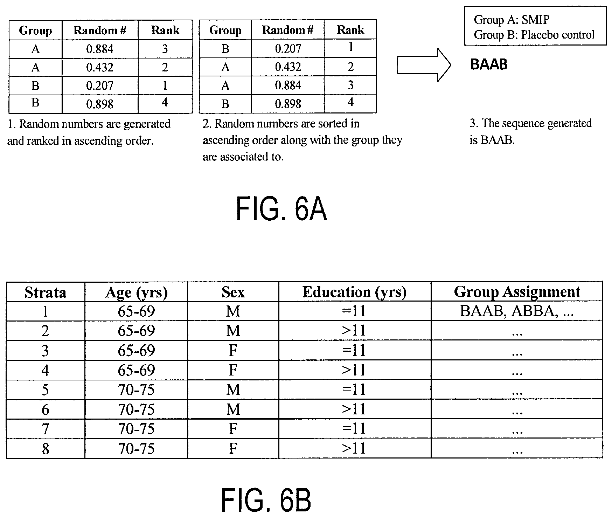

In an embodiment, MCI patients and healthy controls were part of one of two groups: the experimental group (SMIP) or the PC group. They were assigned to a group in a random fashion by using a stratified randomization method as shown in FIG. 6. Participants are assigned to a group in a random fashion by using a stratified randomization method (Friedman, Furberg, & DeMets, 1998), with a block size of four so that the number of participants in the two groups is balanced after every set of four participants. Using an Excel function, for example, random numbers between 0 and 1 are generated for each participant in a set of four participants. They are then ranked in ascending order, and assigned a group based on their rank by the computer. Thus, the assignments of participants will be completely unpredictable.

The groups were balanced in terms of sex, age, and education. The randomization process is performed within every combination of these factors (stratum), i.e. different group assignment sequences are generated for each stratum. Thus, within each clinical group (MCI and healthy older adults), participants of each of the four categories (Women vs. Men, High-Educated vs. Low-educated) are randomly assigned to the training or the control condition. Based on Statistics Canada's criterion, an individual is considered highly educated if he has completed more than 11 years of formal school education.

A table representing the stratified randomization process is shown in FIG. 6B.

A research assistant who is not involved in this specific project is in charge of the randomization process. The assignment takes place when participants are first contacted for a phone interview. The study is double-blind: neither the participant nor the research assistants administering the pre- and post-neuropsychological transfer tests knows which group the participant is assigned to. The only person who has this information is the research assistant administering the training. Extra precaution is taken so that no information about the training sessions is divulged, since the laboratory environment is shared by both the person in charge of training and research assistants. Moreover, participants are asked not to talk about any matter pertaining to their training to other research members in the laboratory.

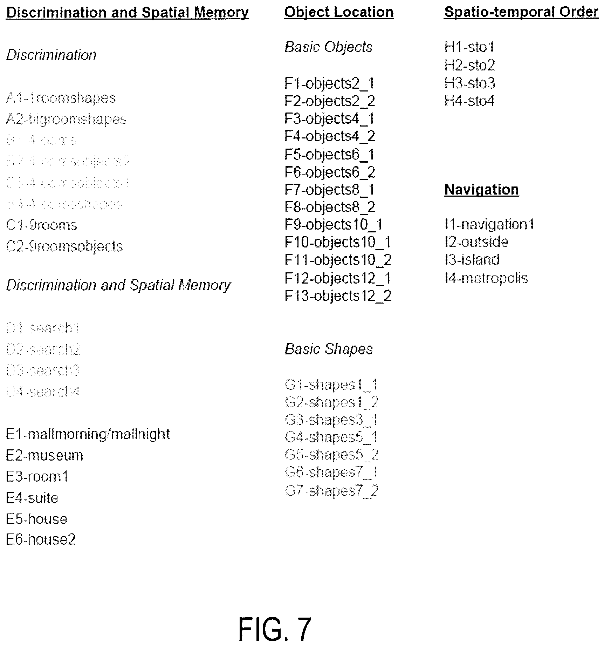

Now referring to FIG. 7, shown is a list of different VR memory training modules in accordance with an embodiment. In an illustrative experiment, these VR memory training modules were administered in 16 sessions of one hour duration, given across eight weeks.

The virtual tasks that form the training program and the transfer tests described below were constructed using a 3D gaming editor called Unreal Tournament Editor 2003 (UT2003, Epic Games). This gaming editor was selected based on availability to the inventor, and it will be understood by those skill in the art that other 2D graphics, 3D editors or engines could also be used to generate the virtual environment.

The 3D gaming editor allowed the design of realistic 3D virtual environments varying in size from small rooms to complex cities and outdoor landscapes utilizing a rich array of textures. Previous research in rodents and research conducted in the inventor's laboratory shows that healthy individuals shift from spatial to response strategies with increased practice or repetition. Consequently, in order to maintain HPC stimulation, it is critical to have participants train in novel environments in order to prevent stimulus-response based habit learning, which no longer requires the HPC. As such, the inventor spent a number of years developing and validating different virtual environments (see FIG.7 for an illustrative set of 46 different training programs) in which the relative positions of objects, landmarks, or rooms need to be memorized.

In an embodiment, the training program is comprised of 16 one-hour spatial memory training sessions administered to participants twice a week during the course of eight weeks. (It will be appreciated that these sessions could be shorter, or could be taken up as a regular training regime for the rest of one's life to maintain brain fitness, so the spatial memory training sessions are not limited to any length of time). During these sessions, instructors meet with participants individually in a quiet room free of distractions. Participants are seated in front of a computer and are given instructions before starting their tasks. The level of difficulty is adjusted for each participant by starting with very easy tasks (low memory load, smaller region of exploration) and progressing to a more complex level (higher memory load, progressively larger and more complex regions to explore) only when participants reach criteria.

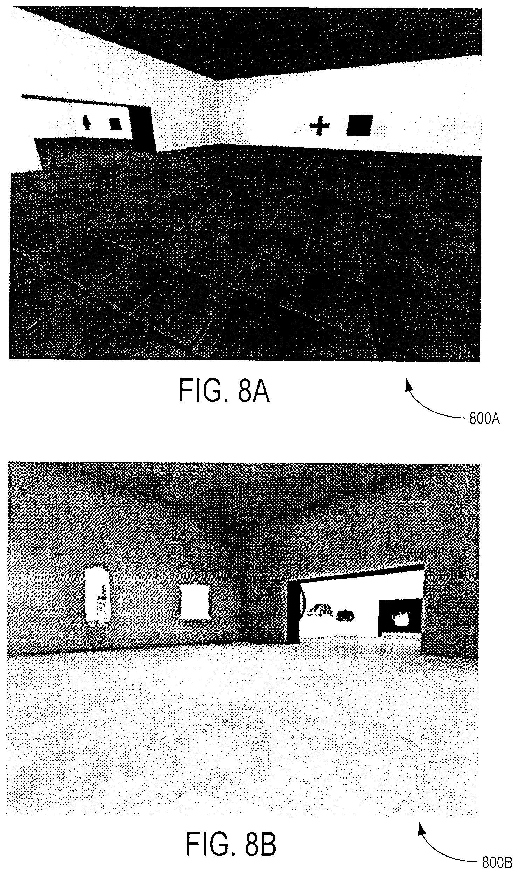

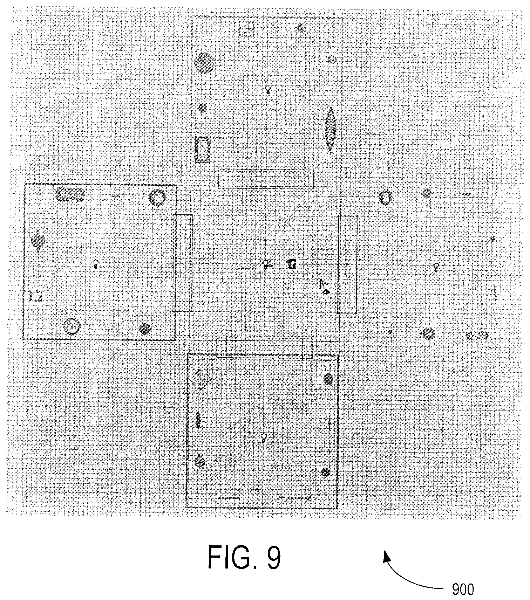

Now referring to FIGS. 8A and 8B, shown are illustrative screenshots taken from selected VR memory training modules for Discrimination tasks. In this training module, participants are asked to search for various shapes (left) or objects (right) from an increasing number of rooms, e.g. "please find the black square in the yellow room" or "find the blue car, in the black room" across eight environments of increasing complexity (number of rooms and objects in the rooms increase). With progress, attentional and cognitive demands increase gradually. Learning is measured in terms of latencies to find target objects. This phase prepares participants for the VR memory training modules for the Discrimination and Spatial Memory phase that follows. FIG. 9 shows an illustrative top view of rooms and a list of objects to find as specified in Table A, below.

**Table A**

| Discrimination Tasks | |
|---|---|
| 4roomsobjects | Latency |
| (Orange room) Pencil | |
| (Red room) 7 up can | |
| (Blue room) Battery | |
| (Red room) Mushroom | |
| (Green room) Shovel | |
| (Blue room) Sword | |
| (Red room) Canoe | |
| (Green room) Chair | |
| (Orange room) Fork | |
| (Blue room) Mug | |
| (Green room) Needle | |

In an embodiment, participants are required to search for and locate shapes or objects (e.g., find the blue car, find the red square) across eight environments of increasing complexity, where the number of rooms and objects in the rooms increase - see FIGS. 8 and 9 for further details.

In another embodiment, participants begin by engaging in the exploration of a realistic-looking environment. They must locate specific objects or rooms and remember their exact positions. Participants are asked to reproduce a top view of the environment including either the objects in it or the layout of its rooms. Remembering the relative positions of objects in a room, from a different perspective, was previously proven to require the HPC.

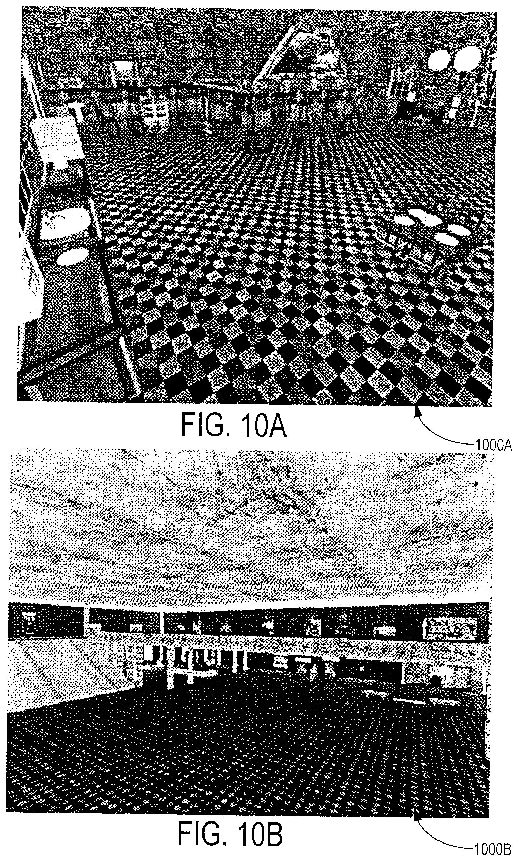

FIGS. 10A and 10B show illustrative screen shots taken from VR memory training modules for Discrimination and Spatial Memory tasks. In this illustrative example, participants must locate objects or rooms and remember their exact position. As participants progress through the tasks, the memory load and difficulty increases across 10 different environments. As the participants progress, they are presented with an increasing number of objects, more complex environments or more complex list of instructions to complete sequentially. Participants are asked to either reproduce a top view of the environment (including the objects in it or the relative position of rooms) or follow a set of instructions concerning things to do in the rooms. Trials are given until participants place all objects in their correct position or until they reach a maximum of trials. Learning is measured in terms of errors in placing objects and latencies to the target objects and locations.

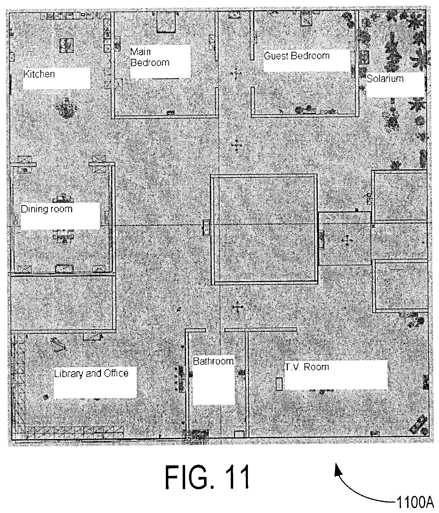

In another embodiment, participants are placed in a room and presented with an array of objects placed on a table. Participants are instructed to examine and learn the precise location of these objects as viewed from all four sides of the table. Remembering positions of objects on a table has previously proven to require the HPC. FIG. 11 shows a top view of a suitable virtual environment. Table B below provides an illustrative example of a list of instructions for participants to follow.

**Table B**

| List of Instructions |
| --- |
| 1. You've just come home. |
| 2. Put your keys on the table in front of you. |
| 3. Hang up your jacket in the closet to your right. |
| 4. Go to the KITCHEN and get yourself some milk. |
| 5. Head to the LIVING ROOM and turn on the television. |
| 6. Check on your mushroom collection in the GREENHOUSE. |
| 7. Pick up one of the books on the small table in the LIBRARY. |
| 8. Fix yourself a toast in the KITCHEN. |
| 9. Find the sewing kit in the YELLOW BEDROOM. |
| 10. Repair the top hat in the MAIN ENTRANCE. |
| 11. Wash your hands in the BATHROOM with the soap on the sink. |
| 12. Find the seat without a plate or glass in the DINING ROOM. |
| 13. Feed the fish in the aquarium. |
| 14. Play some music on the piano in the LIBRARY. |
| 15. Check on the fireplace in the LIVING ROOM. |
| 16. Turn on the fan in the RED BEDROOM. |
| 17. Go to bed in the YELLOW BEDROOM. |

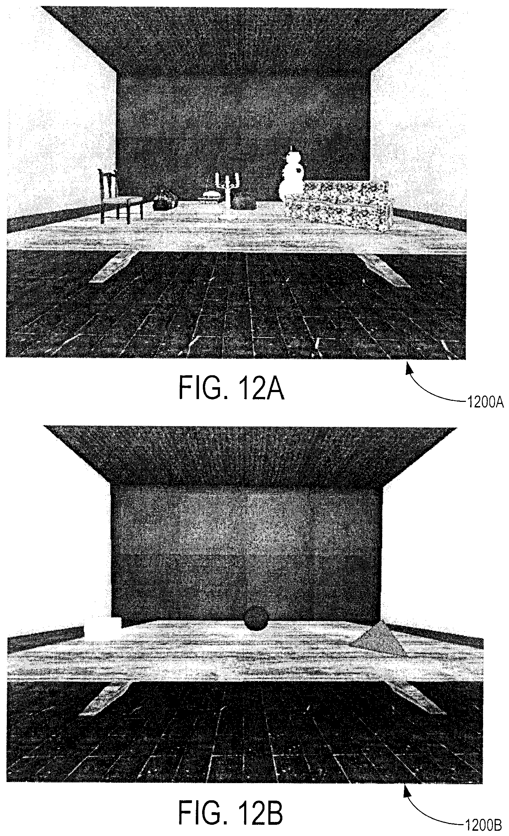

FIGS. 12A and 12B show illustrative screenshots of a VR memory training module for Object Location tasks. Here, participants must remember the location of objects (top) or shapes (bottom) placed on a table. Participants are then asked to reproduce a top view of the objects on the frame of the table provided to the participants. Trials are given until participants place all objects in their correct position or until a maximum of four trials is reached. The number of objects or shapes increases as participants progress through the tasks. Learning is measured in terms of errors, as determined by the difference in distance between an object's actual and observed location. The memory load and difficulty increases across 20 different virtual environments.

In an embodiment, the maximum number of trials for any given VR memory training module has been selected to be four, such that repetition does not lead to the participants relying less on using the HPC region of their brains and more on the CN region. While a maximum of four trials is preferred, setting a maximum of two to six trials may still have the desired effect of focussing on the HPC region for exercise.

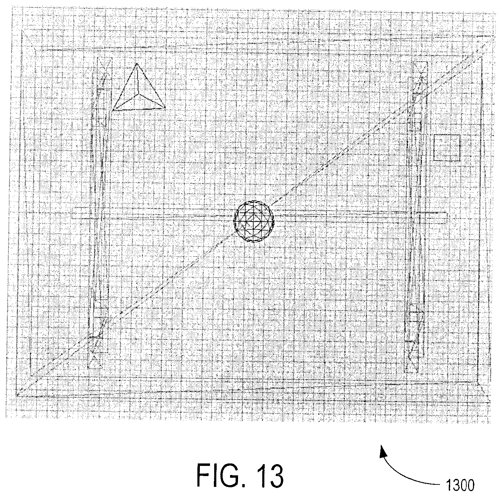

Now referring to FIG. 13, shown is an illustrative example of a top view of a score sheet and a list of objects including: 1. A blue ball; 2. A red triangle; and 3. A yellow square, to place in the Object Location tasks. This task measures the participant's ability to make a mental map of the space that includes all the elements in that space (e.g. the objects) and their relationships. This activity taps directly into the embodiment of using a spatial memory strategy.

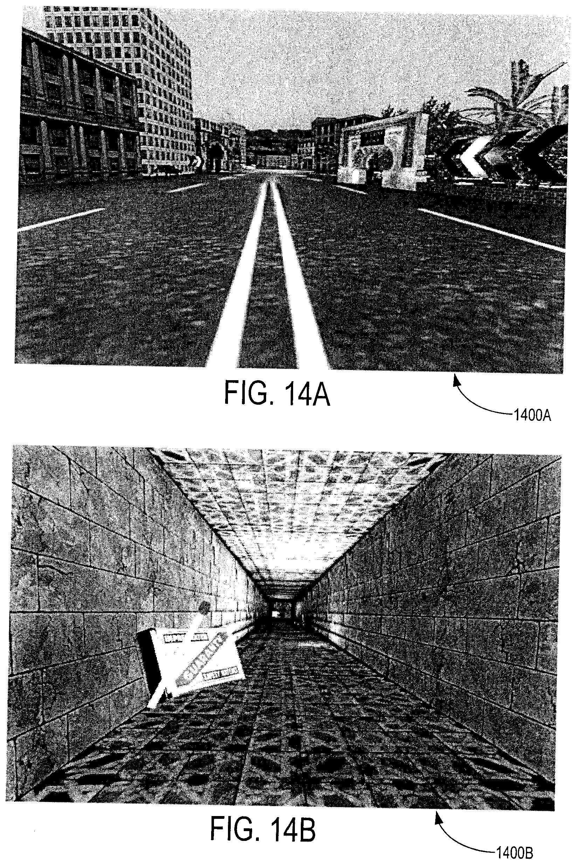

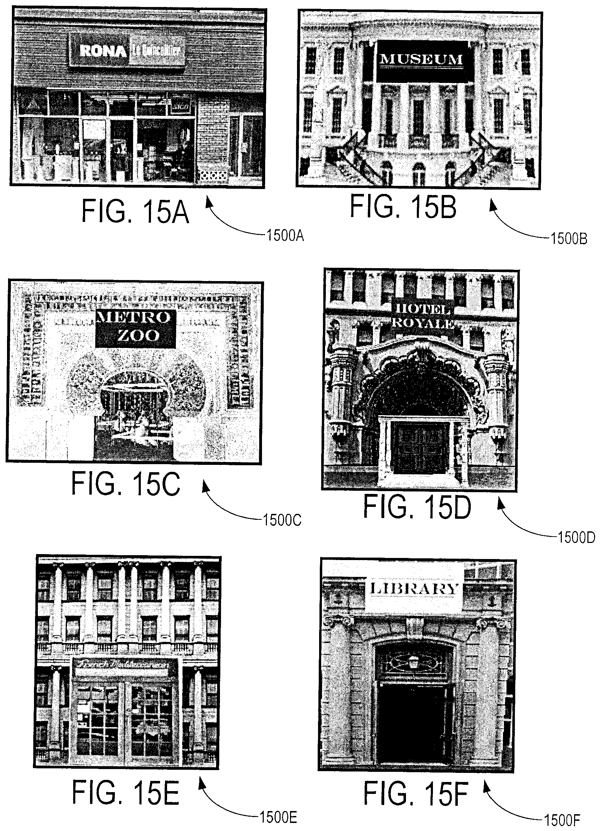

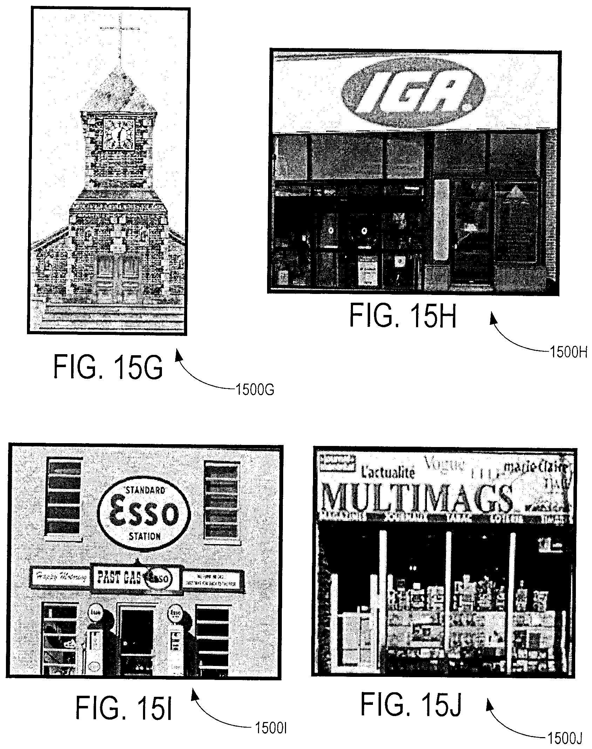

In another embodiment, the ability of participants to remember a sequence of previously seen objects and locations is examined (i.e. memory for temporal order, a component of memory that is dependent on the HPC). Remembering when an event was experienced was previously proven to require the HPC. FIGS. 14A and 14B, shown are illustrative screenshots of a VR memory training module for spatio-temporal order tasks. Here, participants travel along a predetermined path from which objects or locations are visible (e.g., pencil, shovel, church, zoo). While travelling along a straight path, participants must remember the objects or locations in the order that they were presented to the participant. Trials are given until participants list all objects or locations in their correct temporal order or until a maximum of four trials is reached. Learning is measured in terms of errors. The memory load and difficulty increases with number of objects to remember across four environments. In another example, FIGS. 15A - 15J show illustrative screen shots of a VR memory training module for placing landmarks in temporal order in the spatio-temporal order tasks.

In another embodiment, participants explore environments ranging in size from a small village to a large urban landscape that contain multiple landmarks. Following a 20- to 30-minute exploration, participants must reach target locations (e.g. a movie theatre) and remember their position respective to other landmarks within the environment.

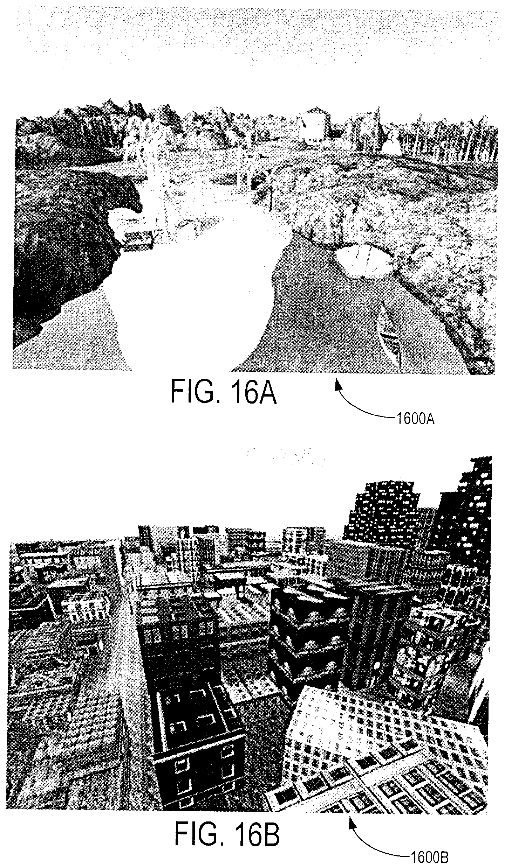

Remembering the positions of landmarks in a virtual town was previously proven to require the HPC. FIGS. 16A and 16B show illustrative screenshots taken from VR memory training modules for navigation tasks. Participants learn the location of objects or landmarks while exploring environments. The size of the environments to be explored increases as participants progress successfully through the tasks.

Additional Training Modules

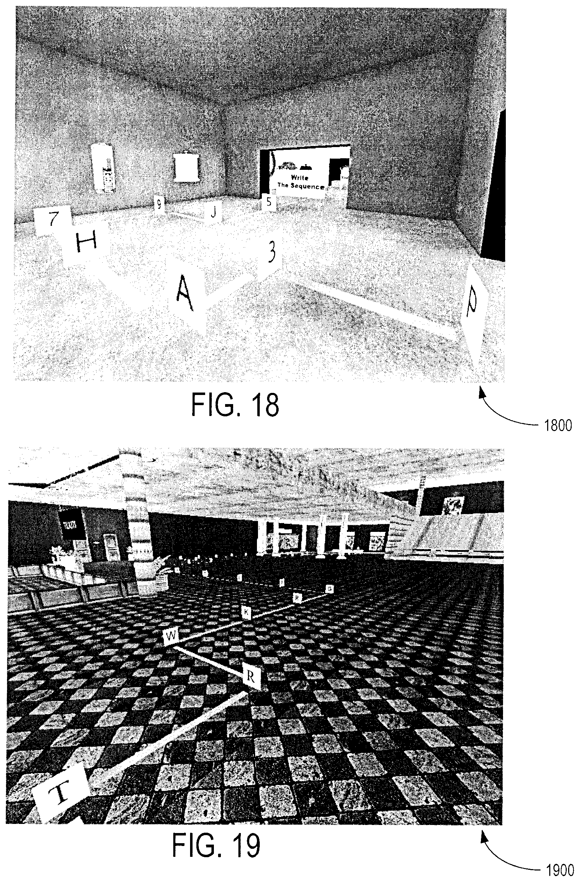

Participants may be encouraged to train their short-term memory by occupying their attention with working memory (WM) demands such as counting backwards by 3 from 1000. Based on these results, the inventor proposes a variety of WM tasks that may activate regions of the frontal lobe. These WM tasks use the same virtual environments as the SMIP to control for the visuo-motor demands of the training, and consists of the same number of sessions and same task durations as the SMIP. As an illustrative example, participants are presented with five types of WM tasks as shown in FIGS.18 - 22.

In an embodiment, participants are required to keep track and to subsequently repeat a sequence of numbers and letters. This task is widely accepted as a measure of WM and WM capacity. In an earlier study, the performance of an auditory NLS task was associated with activation in areas of the brain previously linked to WM, namely the premotor cortex, orbital frontal cortex, dorsolateral prefrontal cortex, and posterior parietal cortex. In the present training module, participants are asked to follow a yellow line through the rooms. Along the line are panels with either a number or letter. As shown in FIG. 18, participants are asked to follow the yellow line through the rooms. Along the line are panels with either a number or letter. When the participants touch the panel, the panel disappears and the next one appears. Participants are asked to remember the sequence as they go along and when they reach the end of the room, they are asked to write down the whole sequence in order of presentation (e.g. P3AH79J5). If the participants make a mistake in the sequence, they are asked to redo that specific room until the correct sequence is learned. The task requires the use of working memory rather than spatial memory and performance is measured by trials to criteria. This task can be made easier when necessary (e.g. for MCI patients) by reducing the number of panels to remember.

In another embodiment, participants are asked to follow a yellow line through the environment. Along the line are panels with a letter from A to Z in random order. When the participants touch a panel, the panel disappears and the next one appears. As shown in FIG. 19, participants are asked to follow the yellow line through the museum. Along the line are panels with a letter from A to Z. Participants are asked to signal when a letter presented is the same as the letter presented 1, 2, or 3 panels before. The task requires the use of working memory. This task can be made easier when necessary (e.g. for MCI patients) by reducing the number of interfering panels or placing several identical panels in a row. A meta-analysis of n-back neuro-imaging studies found consistently robust activation in frontal and parietal cortical regions in participants performing the task. Similar findings confirm that the performance of the n-back task in both men and women results in activation of the superior frontal gyrus, middle frontal gyrus, inferior frontal gyrus, and inferior parietal lobule. In addition to NLS and the n-back task, the control task includes three variations on basic addition and subtraction exercises that require WM.

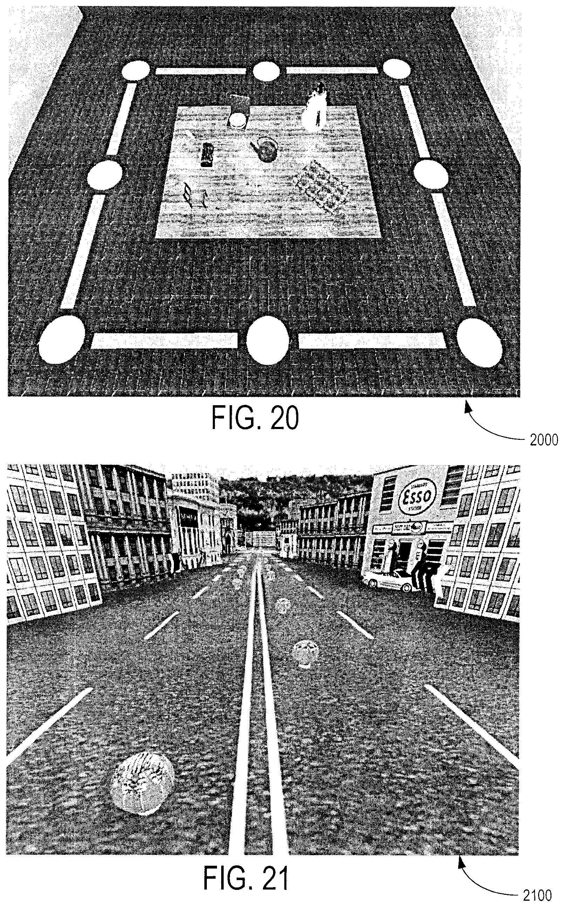

In another embodiment, participants are asked to follow the yellow line around the table clockwise from the start position. Along the line are white circles. Participants are asked to subtract the number three at every circle, starting from the number 1000 (Fig.20). This task can increase in complexity as needed by asking participants to subtract larger numbers as they find white circles. Once the participants have circled the table, they are then asked to circle the table again but this time subtracting four from their last number. The participants are asked to repeat the process again, this time subtracting six, and to give the final number when they are done. The task requires the use of working memory.

In another embodiment, participants are asked to follow the yellow line along the middle of the road or hallway. As shown in FIG. 21, participants are asked to walk down the middle of the road. Starting from the number 100, they are asked to subtract two every time they pass a lantern on the left and add three every time they pass a lantern on the right. The participants are asked to give a final number once they reach the end of the road. The task requires the use of working memory.

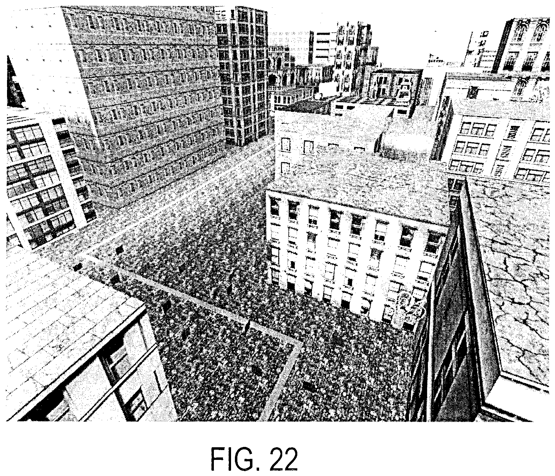

In another embodiment, as shown in FIG. 22, participants are asked follow the yellow line through the town. Along the path are green, red, and yellow panels. As the participants walk along the path, they are asked to keep count of the number of panels of each color. At intervals throughout the town participants are asked to give their total count of each color panel. The task requires the use of working memory. Two of these tasks may be described as dual-tasks, one as a form of task-switching. Such tasks have been shown to activate the dorsolateral prefrontal and parietal cortices.

Alternative Training Modules

In another embodiment, participants are given placebo control training in order to address nonspecific factors related to the SMIP, such as navigation to the laboratory, social interaction with the experimenters, and general cognitive stimulation.

Previous fMRI research indicates that a suitable control for the spatial memory tasks involves a control task that prevents participants from rehearsing spatial relationships by occupying their attention with a task. This kind of control task does not lead to activity in the HPC, even when it is based in a virtual environment. Based on these results, an "educational training placebo control" was modeled from studies in the literature. This control consists of the same number of sessions and same task durations as the SMIP. It involves a learning-based training approach in which participants use computers to view DVD educational programs on nature, cultures, and science.

In each of the 16 one-hour sessions, participants watch a 50-minute program. After watching the video, participants complete written quizzes. These quizzes involve questions relating to the content knowledge presented by the DVD in that session. This protocol uses audio-visual stimulation presented on a computer, as the SMIP, which controls for the visual attentional demands of the training. This protocol follows the successful placebo control task used previously. For example, it was shown that the performance of participants in the placebo control and the no-contact control group (NCC) were equivalent. Participants who underwent the experimental training condition showed significantly greater improvements on cognitive measures compared to those who did the placebo control condition.

**Table C**

| Experimental Design | |
|---|---|
| Experimental | Placebo Control |
| Healthy: Sessions 1 to 4 | Healthy: Sessions 1 to 4 |
| MCI: Sessions 1 - 4 including MRI | MCI : Sessions 1 -4 including MRI |
| Transfer Tests Balanced for order of administration, time of day (AM vs PM) that participants get tested in each group | |
| Session 1: Neuropsychological tests, 4/8VM, Wayfinding in the Virtual Town | Session 1: Neuropsychological tests, 4/8VM, Wayfinding in the Virtual Town |
| Session 2: Neuropsychological tests, self-administered questionnaires | Session 2: Neuropsychological tests, self-administered questionnaires |
| Session 3: Mock Scanning Session. | Session 3: Mock Scanning Session. |
| Task administered: Go/No-Go | Task administered: Go/No-Go |
| Session 4: fMRI scan - task administered: Concurrent Spatial Discrimination Learning Task | Session 4: fMRI scan - task administered: Concurrent Spatial Discrimination Learning Task |
| Training | |
| 16 one-hour Spatial, Temporal and Working Memory Improvement | 16 one-hour Placebo Control |
| Transfer Tests Different versions of all virtual navigation tests and Neuropsychological tests are provided, balanced for the version administered before or after training | |
| Session 1: Neuropsychological tests, 4/8VM, Wayfinding in the Virtual Town | Session 1: Neuropsychological tests, 4/8VM, Wayfinding in the Virtual Town |
| Session 2: Neuropsychological tests, self-administered questionnaires | Session 2: Neuropsychological tests, self-administered questionnaires |
| Session 3: Mock Scanning Session. | Session 3: Mock Scanning Session. |
| Task administered: Go/No-Go | Task administered: Go/No-Go |
| Session 4: fMRI scan - task administered: Concurrent Spatial Discrimination Learning Task | Session 4: fMRI scan - task administered: Concurrent Spatial Discrimination Learning Task |

**Table D**

| |
|---|
| 1. Small Park |
| 2. Seaview Mall |
| 3. Taxi Center |
| 4. Louie's Restaurant |

(continued)

| 5. Ewe & Lamb Restaurant |
| --- |
| 6. Mulliqan's Pub |
| 7. Multimags |
| 8. Kirin Restaurant |
| 9. Newspaper Shop |
| 10. Dollarama |
| 11. Desjardins Bank |
| 12. Big Burger |
| 13. IGA |

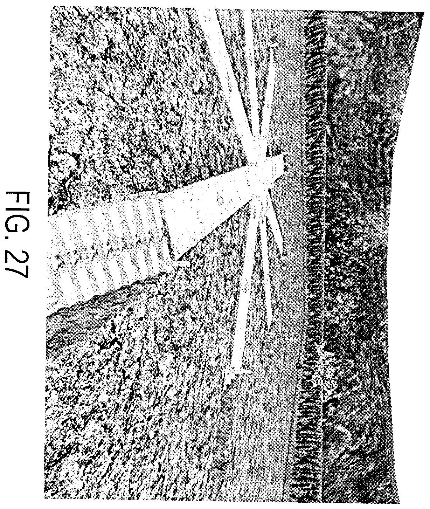

In another embodiment, a 4/8VM computerized task is used to investigate spontaneous strategies used by participants and also to investigate the impact of SMIP on acquisition of the task in terms of errors and time it takes to complete the task. Participants have to find four hidden objects in an eight-arm radial-maze, as described further below. Participants are trained to criterion, ensuring learning in all participants. As shown in FIG. 27, in Part 1, participants retrieve 4 objects at the end of 4 available paths out of 8 that extended from a central platform. In Part 2, participants remember which paths they had already visited and avoid these in order to find the remaining 4 objects. Landmarks surrounding the maze apparatus provide orientation cues. Task is used to dissociate strategies: spatial vs. response with the use of a probe trial during which all landmarks are removed and a wall is raised in order to hide the landscape. Only the participants who learned to find the objects with a response strategy (sequence of right or left turns from a single starting position) perform well. People who learned the location of target objects with respect to landmarks make errors on the probe trial. Therefore, the probe trial is used to dissociate between spatial and response strategies. Measures of learning include reference memory errors (i.e. going into the wrong path for the first time), working memory errors (i.e. entries into a previously visited path), and latencies.

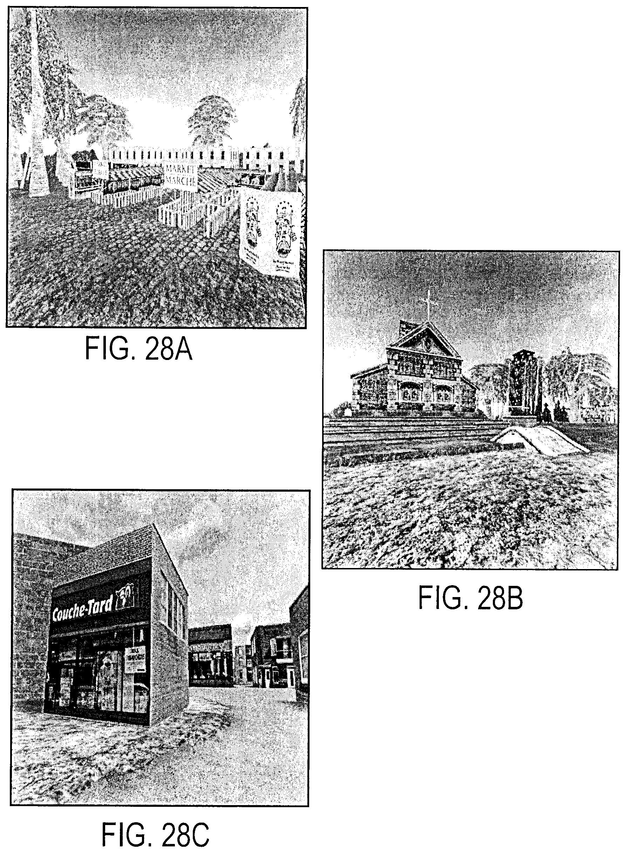

In another embodiment, participants explore a town containing eight landmarks, as shown in FIG. 28, such as a pool, a retail shop, a cinema, etc., for 20 min during which the experimenter verifies that each landmark of the virtual town has been visited at least twice. This is followed by six trials wherein the participant begins at one of the eight landmarks and is asked to reach a particular target using the shortest possible route. The ability to generate a direct route is an indication of spatial learning abilities based on a cognitive map that is formed in the 20 min. exploration phase. The deviation of the route taken from the shortest possible route is the dependent variable. Measures of learning include path lengths and latencies to target locations.

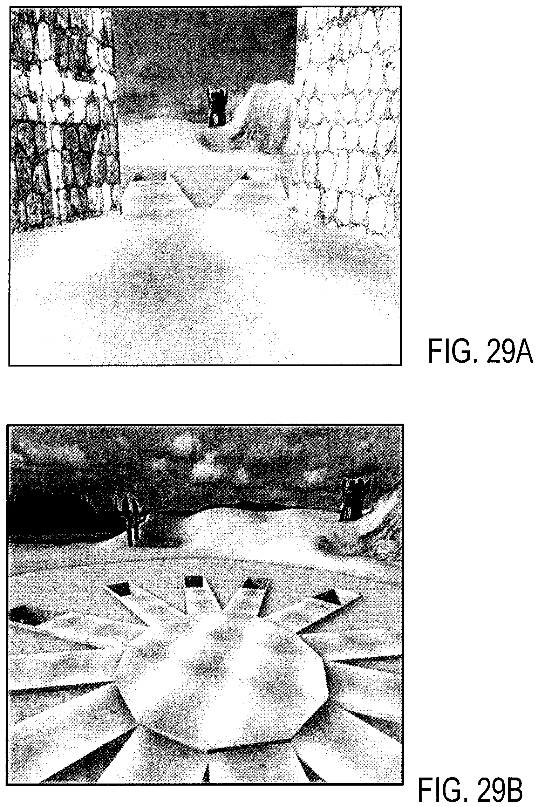

In another embodiment, a go/no-go task consisting of three parts is administered during a practice "Mock" MRI scanning session in order to allow participants to practice lying still and to reduce exclusion rates due to motion artefacts. In the first part, participants are presented with six pathways one by one, three of which contain an object. Upon the fourth presentation, participants are given the choice between entering and not entering each of the six pathways. This step ensures that participants have learnt which pathways contain an object and which are empty. In the second part, the previous pathways are presented in pairs, as shown in FIGS. 29A - 29C. In Part 1 shown in FIG. 29A, participants visit 6 pathways, one by one, 3 of which contain an object. Upon the 4th presentation, participants choose to enter into a pathway or not if they do not believe it contains an object. In Part 2 shown in FIG. 29B, pairs of pathways are presented (top figure). Participants have to choose the pathway containing an object. This part dissociates the ability to use hippocampal dependent spatial learning from response learning. In this part, people who learned the location of target objects using a response strategy (e.g. when I see the tower, take the left pathway) will make errors (because upon learning in Part 1, the target object was also located to the left of the tower). However, people who learned the spatial relationship between the pathway containing the target object and the environment, will not make errors (e.g. I remember that this particular pathway did not contain the object, so I choose the other one (on the right)). Finally, in Part 3 shown in FIG. 29C, all pathways are presented, participants must locate all objects (lower figure showing the maze from a birds eye view that is never seen by the participant from this perspective). Within each of these pairs, one pathway contains an object and the other is empty. Participants have to choose the pathway containing an object. This part dissociates HPC-dependent relational learning from non-relational learning. Learning is measured in terms of latencies as well as errors.

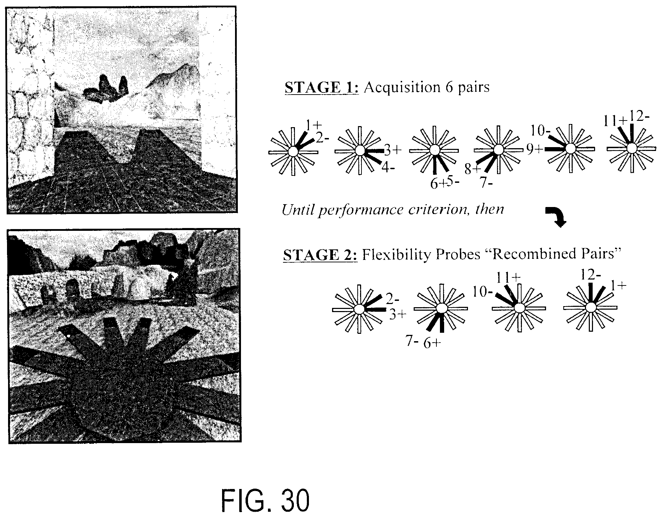

Now referring to FIG. 30, in an embodiment, a Concurrent Spatial Discrimination Learning Task (CSDLT) is presented during an fMRI session as a VR task in which healthy and MCI participants have to find a target object among a pair of arms presented simultaneously, inside a 12-arm radial maze. One of these two arms contains an object located in a pit at the end of the arm, whereas the other arm does not. Participants can learn the position of the arms containing objects by referring to the landscape enhanced with mountains, trees, desert, oasis, surrounding the maze. In the first stage, participants are given multiple trials to learn the location of six objects, presented in six different pairs of arms. The probe trial involves recombining the pairs of arms so that participants are confronted with having to find the object in a novel pair. If the participants remember the position of the object with respect to the environmental landmarks, they will perform well in the probe trials. On the other hand, if participants encoded the position of objects using a response strategy (e.g. when I see the tower, go left) they will perform poorly in the probe trials. This particular task was modeled after a task used in rodents which showed selective impairments in the recombination phase in elderly rodents and which correlated with a reduction in activity of CA3 neurons in a FOS imaging study. In summary, the advantage of these tests over most of the existing spatial memory tests in the literature is that they allow all of the research participants to learn the tasks to criterion, thus controlling for confounding (non-cognitive) changes in affect, motivation, perception, or motor control associated with senescence.

In addition to these tests, the backwards and forward digit span of the WAIS-III, which is a measure of frontal cortex dependent executive function, is used to monitor potential benefits from the SMIP tasks. Altogether, the cognitive battery is distributed in two separate sessions in order to control for fatigue effects. Each session lasts between two and three hours, including resting breaks. In addition, participants undergo a Mock Scanning while performing the go/no-go session and an fMRI scanning session while performing the CSDLT.

Mock Scan with Go/No-Go task: Prior to the two functional and structural scans (before and after the SMIP), participants take part in a mock scanning session with a 0 Tesla scanner. The scanner is used to duplicate the actual scanning experience, including sounds heard and presentation of visual stimuli, but without any exposure to magnetic fields. These mock sessions are used to screen for claustrophobia, proper use of button manipulation, and as a practice session for the actual scan. The Go/No-Go Task, described above, involves learning the location of target objects by exploring pathways presented one at a time. In concurrence with the experimental task, participants must also alternately complete two control tasks, in order to simulate the learning situation of the fMRI scan. Participants are told that they will perform two tasks while in the scanner: the "Experimental" task and the "Random" task (visuo-motor control task). Both tasks are set in different virtual environments. An important difference between the two tasks is that the position of objects can be learned in the experimental task whereas the objects are placed in random arms in the control task. Panels indicating "Experiment" or "Random" are placed in the virtual environments and are presented to the participants for about five seconds at the beginning of each trial.

In another embodiment, participants are asked to navigate in a different environment from the one used in the experimental task. They are asked to retrieve objects in a 12-arm radial maze; however, they are told that it is not possible to predict the location of the objects because they are assigned randomly by the program. In addition, participants are asked to count backwards by 3 from 1000 in order to prevent rehearsal of object locations learned in the experimental trials. This control task is identical to the experimental task in terms of its visual and motor components, differing only in the mnemonic demands of object locations. It is therefore a very efficient control task that successfully isolated HPC and CN activity in a previous protocol.

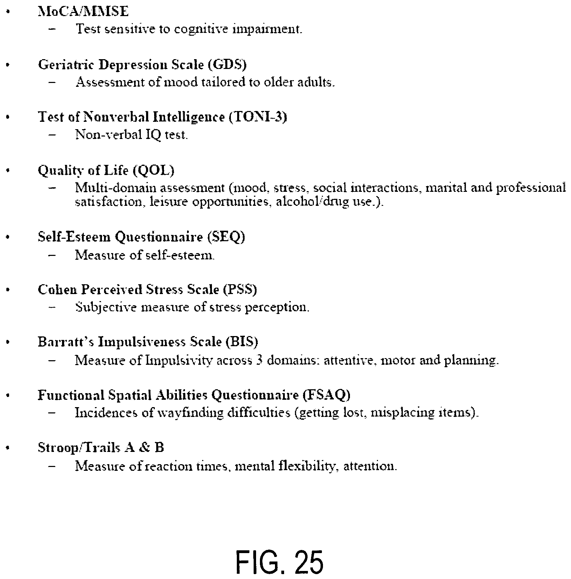

The various transfer tests that may be conducted to test the effectiveness of the memory training, as described above with reference to FIGS. 25 - 30 for example, are optional and may not necessary need to be used.

Results

After completing the VR memory training modules as described above, the participants were tested for improvements in their spatial memory attributable to the training focussing on exercising the HPC region of the brain. The calculations for determining the percentage improvements may be summarized as follows:

PI: Percent Improvements:

$$PI = AI/Average\ (et1,\ et2,\ ct1,\ ct2)*100$$

AI: Absolute Improvements:

$$AI = (Average\ (et1n - et2n)) - (Average\ (ct1n - ct2n))$$

et1: Experimental Transfer 1
et2: Experimental Transfer 2
ct1: Control Transfer 1

ct2: Control Transfer 2
n1: subject 1, n2: subject 2...

In the above calculations, the Absolute Improvement scores do not allow for comparisons between tasks, as they represented values which differed in scale and units of measure. For example, the MoCA was measured by adding scores, whereas the Wayfinding task was measured as path lengths and latencies. As such, the averages of each task may be pooled for both groups and both time points and divided the AI by this average pool to obtain a Percent Improvement (PI) representing a measure of improvement comparable across tasks regardless of units of measure or scale they reflected.

Upon analysing the results of the training activity, it was found that there were significant improvements specific to spatial memory in the experimental group only. Participant demographics and overall cognitive function presented in Table 1 show that the experimental and control groups were similar. Tables 2-3 show results displayed in terms of percent improvement so that comparisons can be made from test to test.

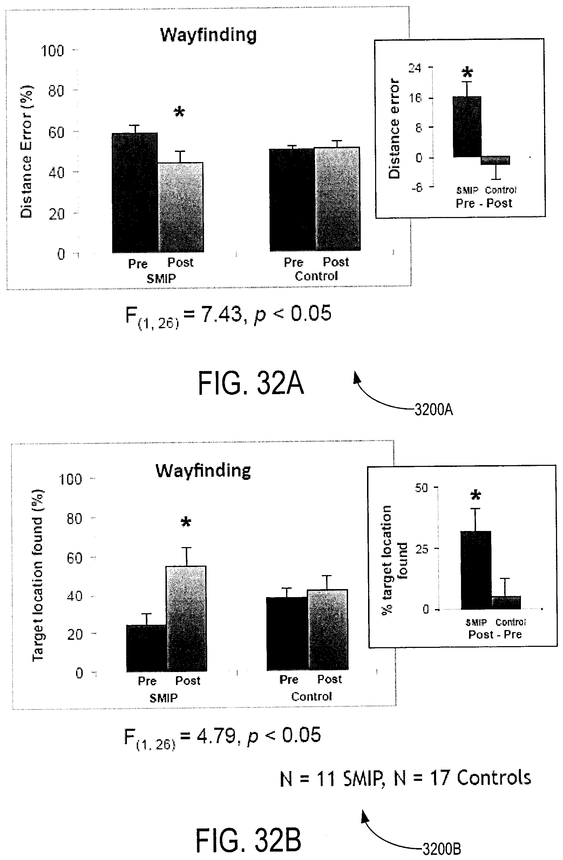

Improvements observed were calculated relative to the performance of the placebo control group (PC group will be used in the future). Importantly, there were no improvements on neuropsychological tests of verbal memory and executive function demonstrating the specificity of the SMIP for spatial memory. A paired t-test (calculated on RANKS due to the low sample size) revealed a significant SMIP effect in lowering errors on the 4/8VM [t=4.64, p<0.001], shortening routes to attain specific target locations in the wayfinding task [t=2.94, p<0.01], and better recall on the ROCF [t=-2.36, p<0.05] (see FIGS.32A and 32B).

Now referring to FIG. 32A, shown are illustrative pre- and post- VR memory training graphs based on percent mean distance error on the Wayfinding task for Experimental and Control groups. Here, percent mean distance error represents the extra distance traveled compared to shortest distance needed to reach goal location. Bars indicate the standard error of the mean (SEM).

Now referring to FIG. 32B, shown are illustrative pre- and post- VR memory training graphs for a Delayed Recall score (30min delay) on the Rey-Osterrieth Complex Figures test for Experimental and Control groups. Bars indicate the standard error of the mean (SEM).

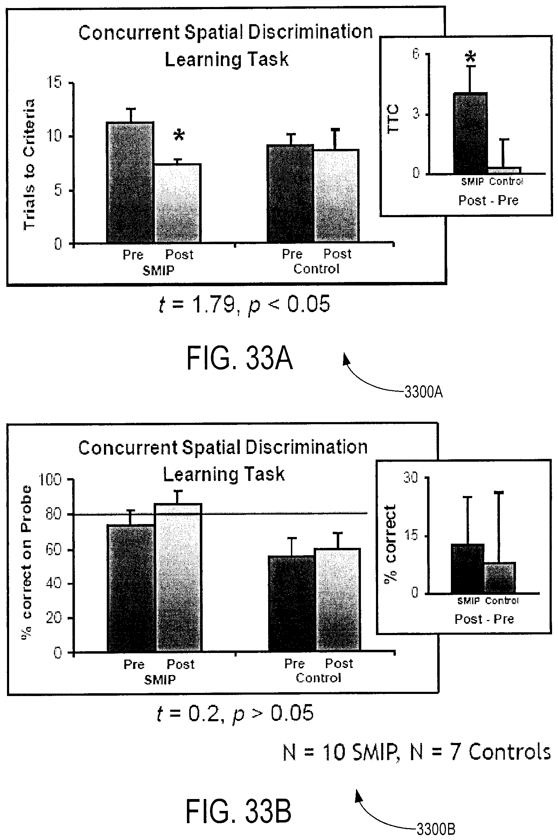

Further, trials to criterion and probe errors on the CSDLT showed significant improvements [t=3.16, p<0.01 and t=-6.08, p<0.000 respectively] (see FIGS.33A and 33B).

FIG. 33A shows illustrative pre- and post- VR memory training graphs for the total number of trials required to reach a specified criteria (11 entries without error out of /12) in the Concurrent Spatial Discrimination Learning Task for Experimental and Control groups. Bars indicate the standard error of the mean (SEM).

FIG. 33B shows illustrative pre- and post- VR memory training graphs for percent correct responses on all probe trials for the Concurrent Spatial Discrimination Learning Task for Experimental and Control groups. Bars indicate the standard error of the mean (SEM).

The fact that the control participants did not exhibit such improvements shows that they were not caused by a mere "learning effect" induced by the repetition of tests. Instead, the improvements found in the experimental group were related to the SMIP and were specific to spatial memory. Additionally, the self-administered questionnaires showed a significant effect of the SMIP in reducing perceived stress [t=-2.52, p<0.05]. This is interesting in the light of results showing that healthy older adults with lower stress, lower cortisol, higher locus of control, and higher self-esteem also have increased grey matter in the HPC. Thus, spatial memory training may increase confidence and reduce stress related to everyday navigation, as testified by the participants in the study, and this may in turn lead to increased HPC grey matter.

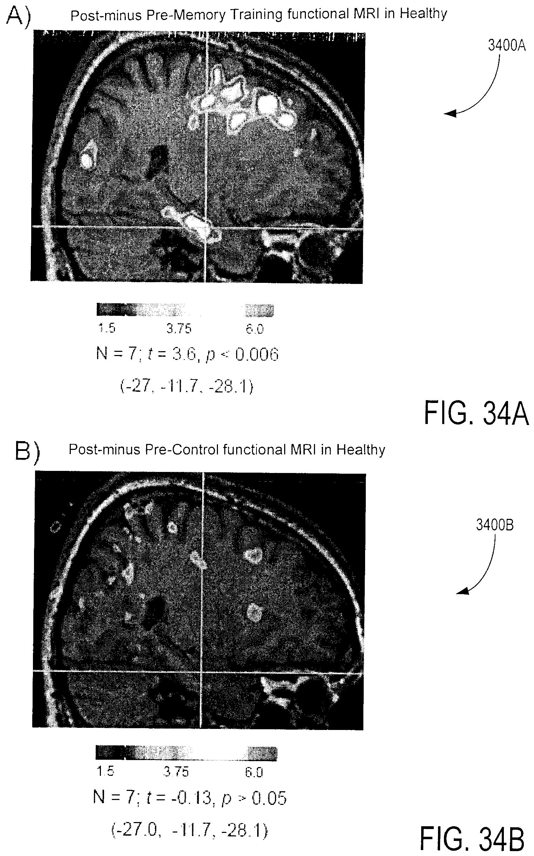

Now referring to FIGS. 34A and 34B show an illustrative contrast between post-training functional MRI scans against pre-training functional MRI scans of healthy older adult participants in (A) the spatial memory improvement program (SMIP) group and (B) controls. The figures show a more extensive increase in activation in the brain in the group that received the SMIP as compared to controls. In particular, the experimental SMIP group shows increases in fMRI activity in the HPC from pre to post-memory training, as well as increases in activity in other areas of cortex after training, whereas the control group shows no such changes in the HPC or elsewhere.

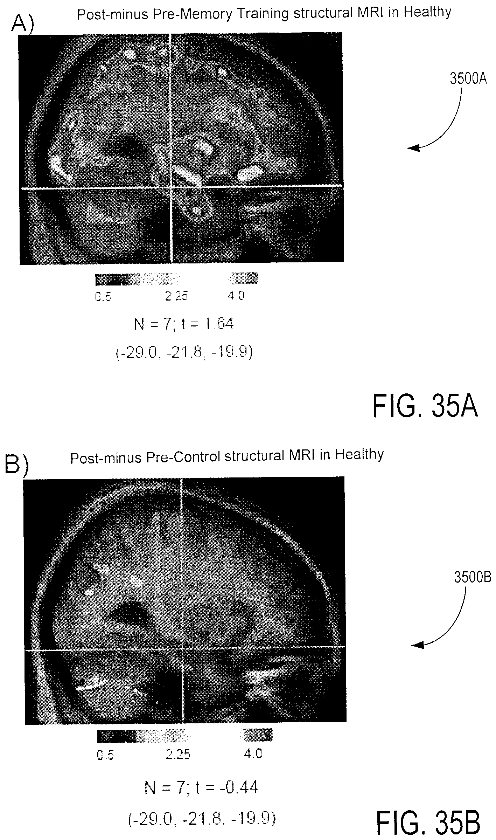

The SMIP led to increased HPC grey matter in the experimental group. Pre- and post-SMIP MRI scans were contrasted. A visible growth in the HPC can be observed in the experimental but not the control group, as shown in FIGS. 35A and 35B (t=1.64, p < 0.05, uncorrected). Now referring to FIGS. 35A and 35B,Voxel Based Morphometry (VBM) may be used to contrast post-training structural MRI scans against pre-training structural MRI scans of healthy older adult participants in (A) the spatial memory improvement program (SMIP) group and (B) controls. FIG. 35A shows increases in grey matter in the HPC (at the cross hair) and several areas of cortex only in the group that received the SMIP. Importantly, the entorhinal cortex, which is one of the first regions to show Alzheimer's Disease pathology along with the HPC, also showed growth as a result of SMIP. In addition, the SMIP led to increased grey matter in other areas of the brain such as the entorhinal cortex region, the perirhinal cortex region, the parahippocampal cortex region, orbitofrontal cortex region, temporal cortex region, parietal cortex region, occipital cortex region, the frontal cortex region, and the amygdala region. The control group shows no such structural MRI changes between the two scans.

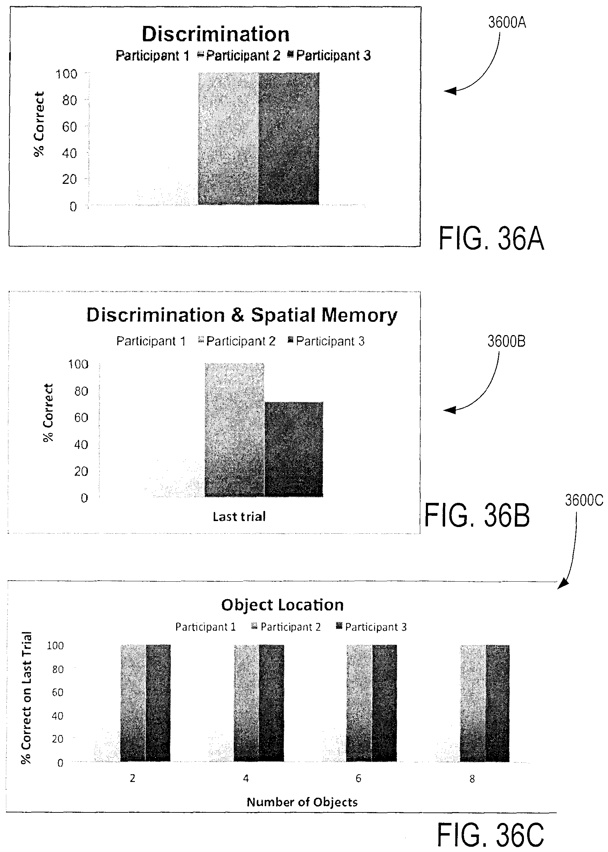

Now referring to FIGS. 36A - 36C, shown are illustrative examples of the performance of three MCI participants in the SMIP group. Percent correct on the Discrimination task. All 3 MCI participants found all of the target objects in the 9 rooms. Percent correct on the Discrimination and Spatial memory during the last trial. Participant 1 reached criteria in 4 trials (TTC = Trials To Criteria) and participant 2 reached criteria in 3 trials. Participant 3 did not reach criteria, however, performance was above 70% on the last trial. Percent correct on last trial of the Object Location task. All participants correctly placed the objects in the 2, 4, 6, and 8 object conditions and reached criteria.

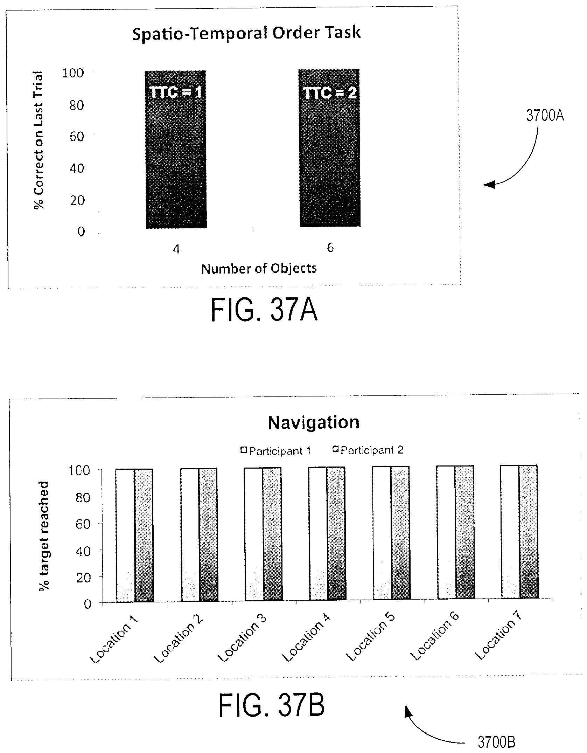

Now referring to FIGS. 37A and 37B, the performance of MCI participants on SMIP are graphed. FIG. 37A shows an average percent correct on last trial of Spatial-Temporal Order task with average number of trials required to reach criteria. All 3 MCI participants recalled the objects in the correct sequence in the 4 objects condition, however, only participants 1 and 2 completed the 6 objects condition. One MCI participant that performed the light version of SMIP only did the 4 object condition. In order to adapt the test to all patients, a "light" version of the tests was used for one experimental (SMIP) and one control MCI (PC) participant. The light version involved the same environments as those described in the methods, but with fewer objects or places to remember. FIG. 37B shows the percent correct at finding target location in the Navigation task. Participant 3 did not receive the island navigation task for lack of time but this participant did complete the navigation task in the small town.

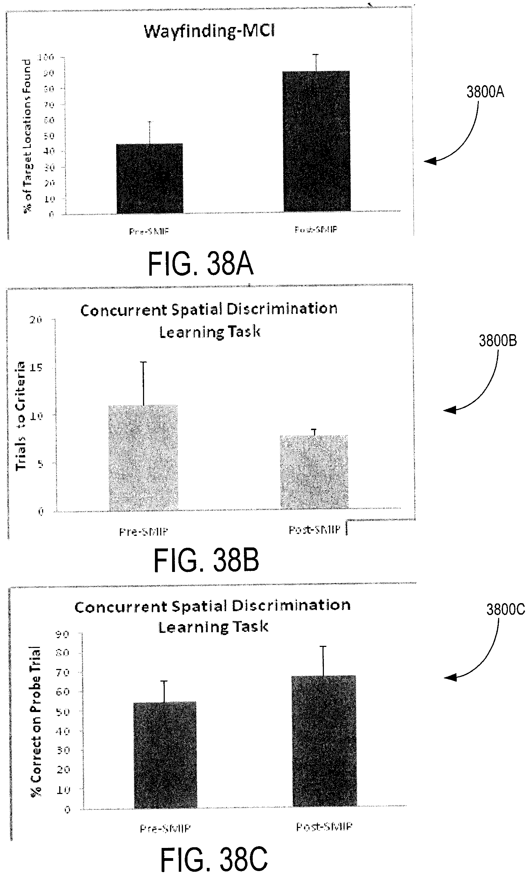

Now referring to FIGS. 38A - 38C, shown are bar graphs of one MCI participant on the Transfer Tests. In FIG. 38A, shown is the percent of target locations found in the Wayfinding task. In contrast to healthy participants who find 100% of the targets, the MCI patient that was tested found 20% target locations in the virtual town during the pre-SMIP testing. The same patient found 100% of the target locations during the post SMIP testing. In FIG. 38B, similar results are observed with the CSDLT and Go/no-go (not shown) where the participant could not reach criteria before SMIP and reached criteria after SMIP. Importantly, in FIG. 38C, the probe trial of the CSDLT indicated that the MCI participant learned the spatial relationship between the target location and environmental landmarks, a process previously shown to require hippocampal function. Subjective benefits showed that the participant greatly appreciated the SMIP: "I feel more hopeful. I feel like I have the tools now. I tell myself to stop, and ask, "Where are you going?"".

Interestingly, WM training-related changes in cortical activity among young adults has shown a correlation between decreases in activation and improved performance on a dual-task69 suggesting an automation of responses. Importantly, these WM tasks are an excellent complement to the SMIP described herein. An easier version of the control task was created to ensure that all MCI participants are able to perform. The feasibility of these working memory tasks was assessed on one MCI participant and results showed that the participant performed above 75% on all tasks.

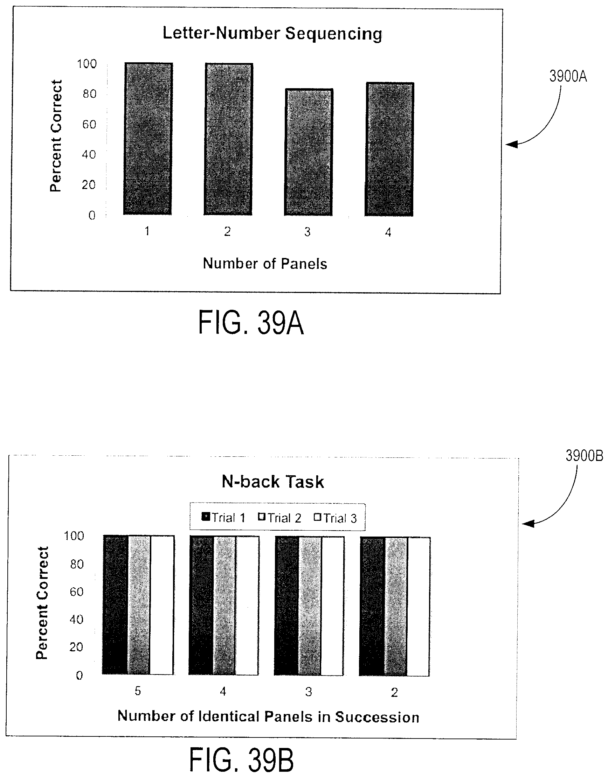

FIGS. 39A and 39B show the performance of one MCI participant on Working Memory tasks. FIG. 39A shows the performance on Letter-Number Sequencing with increasing number of panels and difficulty. The participant remembered a sequence of at least 4 panels at 75% correct performance. FIG. 39B shows the N-back task of N-1 with decreasing numbers of identical panels in succession and increasing difficulty. The participant was able to successfully remember previously seen succession of identical panels when up to 2 panels were presented in succession. These figures show that MCI participants are capable of performing the placebo control tasks.

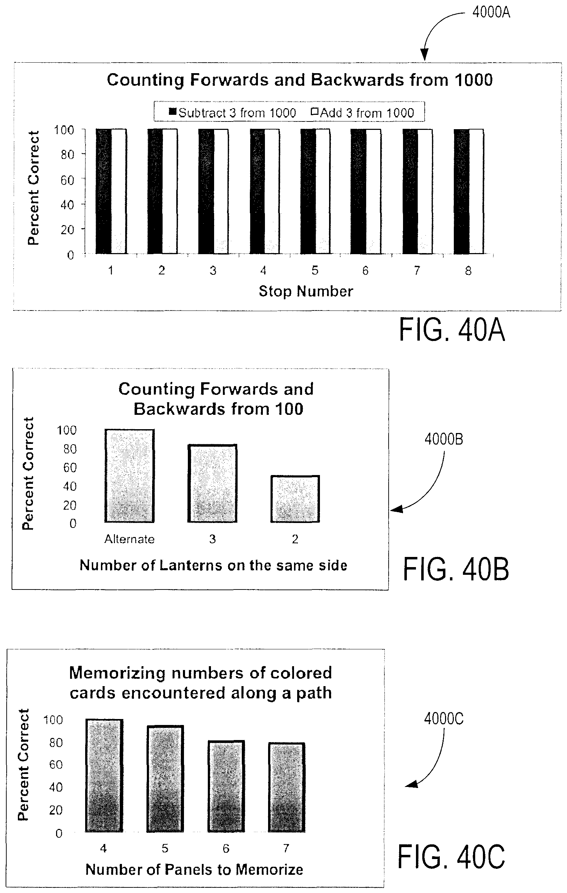

FIGS. 40A - 40C show the performance of one MCI participant on Working Memory tasks. FIG. 40A shows counting backwards and forwards by 3 from 1000 around the table with 8 stops. Participants perform at 100% correct across all levels of difficulty tested. FIG. 40B shows adding and Subtracting 10 from 100 according to location of lantern (left or right). FIG. 40C shows memorizing numbers of color cards encountered along a path with increasing number of panels and difficulty. These figures show that MCI participants are capable of performing the placebo control tasks.

Preliminary results also show that both MCI and healthy participants can successfully achieve the placebo control task with scores above 70% correct confirming feasibility of the placebo control task (Fig. 40B).

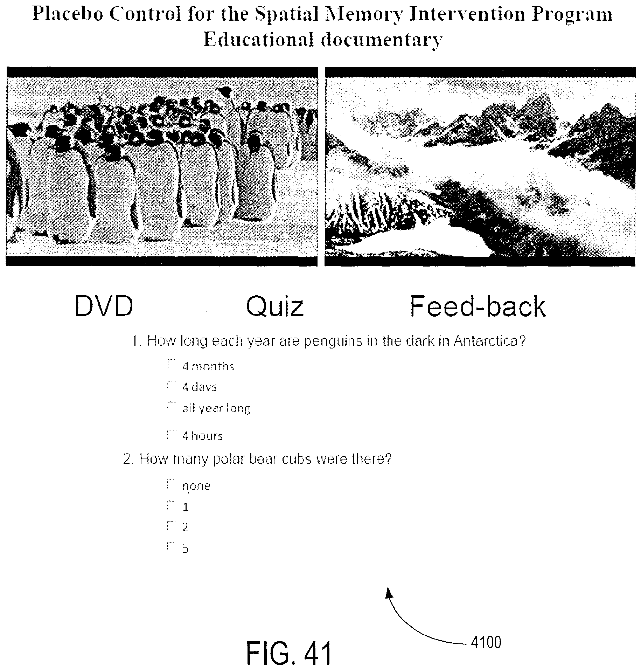

FIG. 41 shows an illustrative example of a placebo control that may optionally be used. By way of example, a placebo task may involve passively viewing a documentary. A participant is asked to carefully watch a 50 min educational DVD about nature, cultures and science, on a computer and he is told that he will have to answer a questionnaire at the end. At the end of the film, he is given a written questionnaire including 10 questions. For each question, he has to choose the correct answer(s) among the four possible choices. The questions vary in level of difficulty and focus on the information that were presented in the film. The experimenter scores the questionnaire. If the participants made some mistakes, the experimenter provides the correct answers with explanation and gives feedback to the participant about the number of errors. The task performance is measured by percentage of correct answer to the questionnaires.

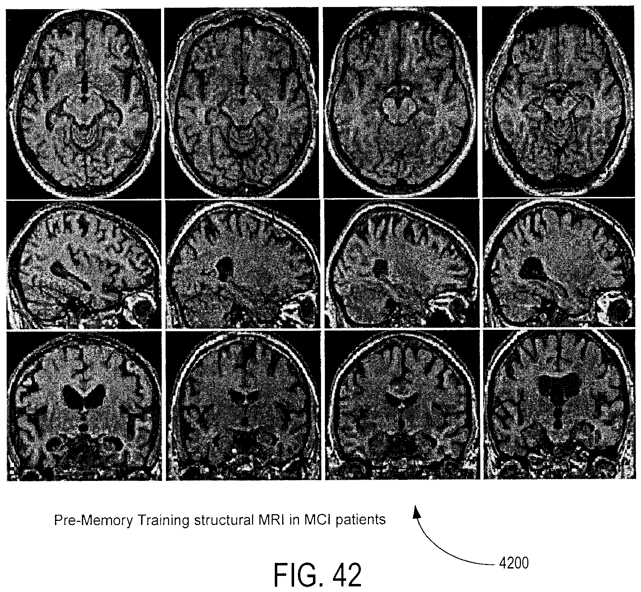

FIG. 42 shows illustrative examples of pre-spatial memory improvement program (SMIP) structural MRI scans of four patients with Mild Cognitive Impairment (MCI). These slides show that patients were successfully scanned with MCI before the SMIP. Based on the preliminary behavioral results, it is expected that an increase in hippocampal grey matter would be shown after the Spatial Memory Improvement Program (SMIP).

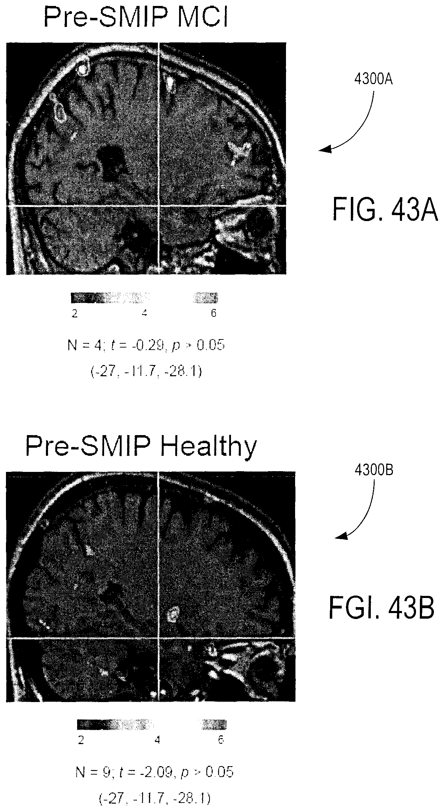

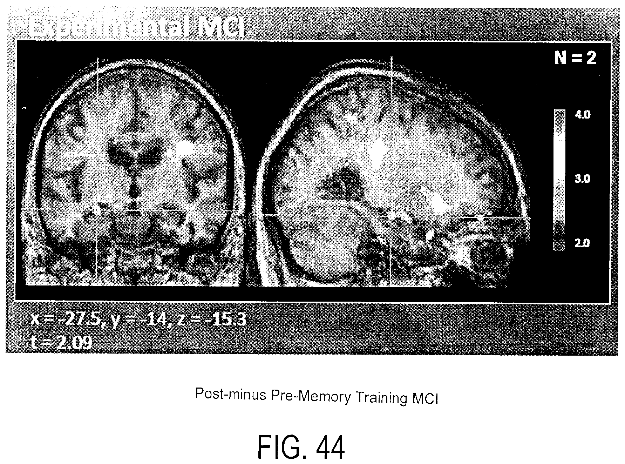

FIGS. 43A and 43B show pre-training functional MRI scans of Mild Cognitive Impairment and Healthy participants, average of first experimental trial. All four MCI participants performed the CSDLT in the scanner. This slide shows the lack of fMRI activity in the HPC during the CSDLT in both groups. Based on the preliminary behavioral results in MCI patients, it is expected that a significant increase in fMRI activity of the HPC would be shown after the Spatial Memory Improvement Program (SMIP) as found in the Healthy participants as depicted in FIG. 44.

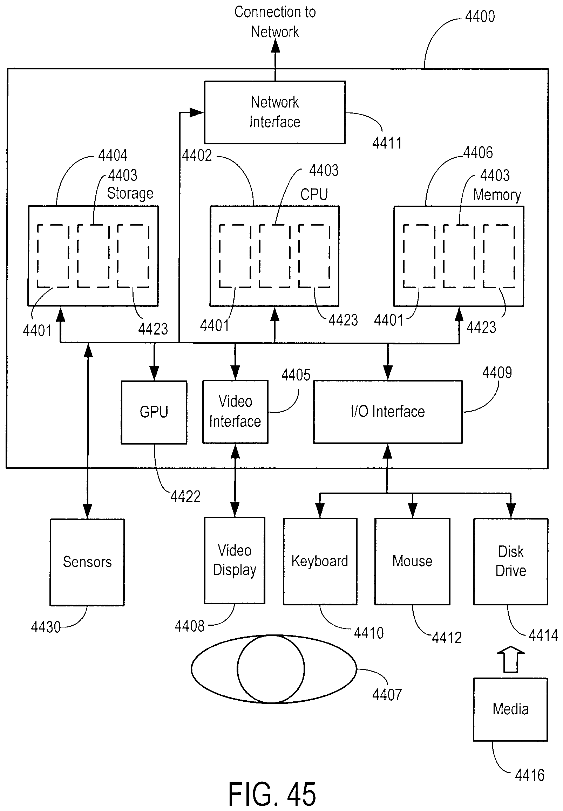

The present system and method may be practiced in various embodiments. A suitably configured computer device, and associated communications networks, devices, software and firmware may provide a platform for enabling one or more embodiments as described above. By way of example, FIG. 45 shows a generic computer device 4400 that may include a central processing unit ("CPU") 4402 connected to a storage unit 4404 and to a random access memory 4406. The CPU 4402 may process an operating system 4401, application program 4403, and data 4423. The operating system 4401, application program 4403, and data 4423 may be stored in storage unit 4404 and loaded into memory 4406, as may be required. Computer device 4400 may further include a graphics processing unit (GPU) 4422 which is operatively connected to CPU 4402 and to memory 4406 to offload intensive image processing calculations from CPU 4402 and run these calculations in parallel with CPU 4402. An operator 4407 may interact with the computer device 4400 using a video display 4408 connected by a video interface 4405, and various input/output devices such as a keyboard 4410, mouse 4412, and disk drive or solid state drive 4414 connected by an I/O interface 4409. In a known manner, the mouse 4412 may be configured to control movement of a cursor in the video display 4408, and to operate various graphical user interface (GUI) controls appearing in the video display 4408 with a mouse button. The disk drive or solid state drive 4414 may be configured to accept computer readable media 4416. The computer device 4400 may form part of a network via a network interface 4411, allowing the computer device 4400 to communicate with other suitably configured data processing systems (not shown). One or more different types of sensors may be used to receive input from various sources.

In an embodiment, operator 4407 may interact with the computer device 4400 using VR goggles 4420 which may be worn over the eyes of the operator 4407 like glasses. By blocking or limiting the peripheral vision of the operator 4407 and presenting an entire field of view display, the VR goggles 4420 may provide a more immersive visual experience. In an embodiment, the VR goggles 4420 may be fitted with an accelerometer or other motion sensor to allow the operator 4407 to navigate through a virtual environment by changing the position of the operator 4407, such as by turning the operator's head, for example.

While the above description provides illustrative examples of one or more systems or methods in accordance with embodiments of the invention, it will be appreciated that other systems or methods may be within the scope of the present invention as claimed below.

**Claims**

1. A computer-implemented system for providing a virtual reality (VR) environment for treatment or prevention of a neurological or psychiatric disorder, in particular Alzheimer, or cognitive deficits in normal aging by stimulating a targeted area of the brain, the system adapted to:

   - access at least one VR memory training module targeting at least one of the hippocampus and neighboring entorhinal cortex region of a participant's brain including one or more memory training tasks comprising interactive user controlled navigation tasks to be performed within a three- dimensional (3D) VR environment;
   - execute the at least one VR memory training module including one or more memory training tasks comprising interactive user controlled navigation tasks configured to train the participant's understanding of relationships between at least one of spatial and temporal details in the 3D VR environment in order to target the at least one of the hippocampus and neighboring entorhinal cortex region of the participant's brain and to display a 3D VR environment including one or more memory training tasks comprising interactive user controlled navigation tasks in order to stimulate and/or promote growth of grey matter in the hippocampus and neighboring entorhinal cortex region of the participant's brain;
   - receive an input from the participant via an interactive controller to assess the participant's understanding of relationships between the at least one of spatial and temporal details in the 3D VR environment in an alternate view; and
   - analyze the results of one or measurements of the participant's brain structure or brain activity before and after, or during the execution of the at least one VR memory training modules for evidence of the stimulation and/or promote growth of grey matter in the hippocampus and neighboring entorhinal cortex region of the participant's brain resulting from the execution of the one or more training tasks, wherein the measurement comprises a structural or functional magnetic resonance imaging (MRI) scan or feedback from sensors indicating the level of brain activity in particular regions of the brain, whereby, the effectiveness of the at least one VR memory training module in targeting the at least one of the hippocampus and neighboring entorhinal cortex region of the participant's brain can be determined; and
   - based on this effectiveness, determine and/or amend the duration and difficulty of the subsequent VR memory training module.

2. The system of claim 1, further comprising:

- a control module configured to access and execute the at least one VR memory training module;
- a VR engine configured to display the VR environment; and
- an interactive navigational controller to receive the input to navigate the 3D VR environment; and
- wherein, the system is further adapted to provide feedback to the participant on how the participant should perform the one or more training tasks comprising interactive user controlled navigation tasks.

3. The system of claim 1 or 2,
wherein the control module is configured to determine which VR memory training module to retrieve and execute in dependence upon the measured effectiveness of a previous VR memory training module training session in targeting at least one of the hippocampus and neighboring entorhinal cortex region of a participant's brain.

4. The system of any one of claims 1 to 3,
wherein the type of memory trained by the one or more memory training tasks comprises one or more of relational memory, episodic memory, semantic memory, declarative memory, temporal memory, spatial memory, spatio-temporal memory, working memory, short-term memory, navigation, and wayfinding.

5. The system of any one of claims 1 to 3,
wherein the selected region of the participant's brain further comprises one or more of the perirhinal cortex region, the parahippocampal cortex region, orbitofrontal cortex region, temporal cortex region, parietal cortex region, occipital cortex region, the frontal cortex region, the amygdala region and the caudate nucleus region.

6. The system of any one of claims 1 to 4,
further comprising one or more VR modules targeting discrimination, attention, perception, dual tasks, and task-switching.

7. The system of any one of claims 1 to 6 for use in a computer-implemented method for providing a 3D virtual reality (VR) environment for the treatment or prevention of a neurological or psychiatric disorder, in particular Alzheimer, or cognitive deficits in normal aging by stimulating a targeted area of the brain.


**Patentansprüche**

1. Computerimplementiertes System zur Bereitstellung einer virtuellen Realitätsumgebung (VR) zur Behandlung oder Verhinderung einer neurologischen oder psychiatrischen Funktionsstörung, insbesondere Alzheimer, oder von kognitiven Defiziten bei normaler Alterung durch Stimulierung eines zielorientierten Areals des Gehirns, wobei das System geeignet ist zum:

- Abrufen mindestens eines VR-Gedächtnistrainingsmoduls, das auf den Hippokampus und/oder das benachbarte entorhinale Hirnrindenareal eines Probandengehirns abzielt, das eine oder mehrere Gedächtnistrainingsaufgaben umfasst, die interaktive, anwendergesteuerte Orientierungsaufgaben einschließen, die innerhalb einer dreidimensionalen-(3D)-VR-Umgebung durchzuführen sind;
- Abarbeiten des mindestens einen VR-Gedächtnistrainingsmoduls, das eine oder mehrere Gedächtnistrainingsaufgaben umfasst, die interaktive, anwendergesteuerte Orientierungsaufgaben einschließen, ausgestaltet zum Trainieren des Probandenverständnisses von Beziehungen zwischen zumindest einem von räumlichen und zeitlichen Details in der dreidimensionalen VR-Umgebung, um auf den Hippokampus und/oder das benachbarte entorhinale Hirnrindenareal des Probandengehirns abzuzielen und eine dreidimensionale VR-Umgebung mit einer oder mehreren Gedächtnistrainingsaufgaben anzuzeigen, die interaktive, anwendergesteuerte Orientierungsaufgaben einschließen, um das Wachstum von grauer Substanz in dem Hippokampus und dem benachbarten entorhinalen Hirnrindenareal des Probandengehirns zu stimulieren und/oder zu unterstützen;
- Empfangen einer Eingabe von dem Probanden über ein interaktives Steuerelement zum Bewerten des Probandenverständnisses von Beziehungen zwischen dem zumindest einen von räumlichen und zeitlichen Details in der dreidimensionalen VR-Umgebung in einer wechselnden Absicht; und
- Analysieren der Ergebnisse einer von Messungen der Gehirnstruktur oder Gehirnaktivität des Probanden vor und nach oder während der Abarbeitung des mindestens einen VR-Gedächtnistrainingsmoduls zum Nachweis der Stimulierung und/oder Förderung des Wachstums von grauer Substanz in dem Hippokampus und dem benachbarten entorhinalen Hirnrindenareal des Probandengehirns, die sich aus der Abarbeitung der einen oder mehreren Trainingsaufgaben ergeben, wobei die Messung eine strukturelle oder funktionelle Kernspintomografieuntersuchung (MRI) oder Rückinformation von Sensoren einschließt, die den Grad der Gehirnaktivität in speziellen Regionen des Gehirns anzeigen, wodurch die Erfolgswirksamkeit des mindestens einen VR-Gedächtnistrainingsmoduls beim Abzielen auf den Hippokampus und/oder das benachbarte entorhinale Hirnrindenareal des Probandengehirns bestimmt werden kann; und
- Bestimmen und/oder Ändern der Dauer und Schwierigkeit des nachfolgenden VR-Gedächtnistrainingsmoduls basierend auf dieser Erfolgswirksamkeit.

2. System nach Anspruch 1, des Weiteren umfassend:

- ein Steuerungsmodul, ausgestaltet zum Abrufen und Abarbeiten des mindestens einen VR-Gedächtnistrainingsmoduls,
- eine VR-Engine, ausgestaltet, um die VR-Umgebung anzuzeigen; und
- ein interaktives Orientierungssteuerelement, um die Eingabe zum Steuern der dreidimensionalen VR-Umgebung zu empfangen; und
- wobei das System des Weiteren angepasst ist, dem Probanden Rückmeldung darüber zu geben, wie der Proband die eine oder mehrere, interaktive, anwendergesteuerte Orientierungsaufgaben einschließende Trainingsaufgaben durchführen sollte.

3. System nach Anspruch 1 oder 2,
wobei das Steuerungsmodul gestaltet ist, zu bestimmen, welches VR-Gedächtnistrainingsmodul abzufragen und abzuarbeiten in Abhängigkeit von der gemessenen Erfolgswirksamkeit einer vorhergehenden Trainingssitzung des VR-Gedächtnistrainingsmoduls beim Abzielen auf den Hippokampus und/oder das benachbarte entorhinale Hirnrindenareal eines Probandengehirns.

4. System nach einem der Ansprüche 1 bis 3,
wobei der durch die eine oder mehrere Gedächtnistrainingsaufgaben trainierte Gedächtnistyp eins oder mehrere von Beziehungsgedächtnis, episodischem Gedächtnis, semantischem Gedächtnis, deklarativem Gedächtnis, zeitlichem Gedächtnis, räumlichem Gedächtnis, räumlichzeitlichem Gedächtnis, Arbeitsgedächtnis, Kurzzeitgedächtnis, Orientierung und Wegfindung einschließt.

5. System nach einem der Ansprüche 1 bis 3,
wobei die ausgewählte Region des Probandengehirns des Weiteren eins oder mehrere von perirhinalem Hirnrindenareal, parahippocampalem Hirnrinden-areal, orbitofrontalem Hirnrindenareal, Temporallappenhirnrindenareal, parietalem Hirnrindenareal, Okzipitalhirnrindenareal, frontalem Hirnrindenareal, Amygdala-Areal und Caudatkernareal einschließt.

6. System nach einem der Ansprüche 1 bis 4,
des Weiteren umfassend ein oder mehrere VR-Module, die auf Unterscheidungsvermögen, Aufmerksamkeit, Sinneswahrnehmung, Doppelaufgaben und Aufgabenwechsel abzielen.

7. System nach einem der Ansprüche 1 bis 6 zur Verwendung in einem computerimplementierten Verfahren zur Bereitstellung einer dreidimensionalen virtuellen Realitätsumgebung (VR) zur Behandlung oder Verhinderung einer neurologischen oder psychiatrischen Funktionsstörung, insbesondere Alzheimer, oder von kognitiven Defiziten bei normaler Alterung durch Stimulierung eines zielorientierten Areals des Gehirns.

**Revendications**

1. Système mis en œuvre par ordinateur destiné à fournir un environnement de réalité virtuelle (VR) pour traiter ou prévenir un dysfonctionnement neurologique ou psychiatrique, en particulier Alzheimer, ou des déficiences cognitives dans un vieillissement normal en stimulant une zone cible du cerveau, le système étant adapté pour :

- accéder au moins à un module d'entraînement de mémoire VR ciblant au moins un élément parmi l'hippocampe et la région du cortex entorhinal voisin du cerveau d'un participant incluant une ou plusieurs tâches d'entraînement de mémoire comprenant des tâches de navigation interactive commandées par utilisateur qu'il s'agit d'exécuter à l'intérieur d'un environnement VR tridimensionnel (3D);

- exécuter ledit au moins un module d'entraînement de mémoire VR incluant une ou plusieurs tâches d'entraînement de mémoire comprenant des tâches de navigation interactive commandées par utilisateur, configurées pour entraîner la compréhension du participant concernant les relations entre au moins un détail parmi un détail spatial et un détail temporel de l'environnement VR 3D afin de cibler ledit au moins un élément parmi l'hippocampe et la région du cortex entorhinal voisin du cerveau du participant, et pour afficher un environnement VR 3D incluant une ou plusieurs tâches d'entraînement de mémoire comprenant des tâches de navigation interactive commandées par utilisateur afin de stimuler et/ou de promouvoir une croissance de matière crise dans l'hippocampe et la région du cortex entorhinal voisin du cerveau du participant ;

- recevoir une saisie par le participant via un contrôleur interactif pour évaluer la compréhension du participant concernant les relations entre lesdits au moins un détail parmi un détail spatial et un détail temporel dans l'environnement VR 3D dans une vue alternative ; et

- analyser les résultats ou les mesurages d'une structure de cerveau du participant ou d'une activité cérébrale avant et après, ou pendant l'exécution desdits au moins un module d'entraînement de mémoire VR pour mettre en évidence la stimulation et/ou la promotion de croissance de matière crise dans l'hippocampe et la région du cortex entorhinal voisin du cerveau du participant résultant de l'exécution desdites une ou plusieurs tâches d'entraînement, dans lequel le mesurage comprend un balayage structurel ou fonctionnel par imagerie à résonance magnétique (MRI) ou un retour provenant de capteurs indiquant le niveau d'activité cérébrale dans des régions particulières du cerveau, permettant ainsi de déterminer l'efficacité dudit au moins un module d'entraînement de mémoire VR pour cibler ledit au moins un élément parmi l'hippocampe et la région du cortex entorhinal voisin du cerveau du participant ; et

- sur la base de cette efficacité, déterminer et/ou modifier la durée et la difficulté du module d'entraînement de mémoire VR suivant.

2. Système selon la revendication 1, comprenant en outre :

- un module de commande configuré pour accéder audit et pour exécuter ledit au moins un module d'entraînement de mémoire VR ;
- un moteur VR configuré pour afficher l'environnement VR ; et
- un contrôleur de navigation interactive pour recevoir la saisie afin de naviguer dans l'environnement VR 3D ; et
- dans lequel le système est en outre adapté pour fournir un retour au participant sur la manière dont le participant devrait exécuter lesdites au moins une ou plusieurs tâches d'entraînement comprenant des tâches de navigation interactive commandées par utilisateur.

3. Système selon la revendication 1 ou 2,
dans lequel le module de commande est configuré pour déterminer quel module d'entraînement de mémoire VR doit être récupéré et exécuté en dépendance de l'efficacité mesurée d'une cession d'entraînement précédente par le module d'entraînement de mémoire VR pour cibler au moins un élément parmi l'hippocampe et la région du cortex entorhinal voisin du cerveau du participant.

4. Système selon l'une quelconque des revendications 1 à 3,
dans lequel le type de mémoire entraîné par lesdites une ou plusieurs tâches d'entraînement de mémoire comprend un ou plusieurs types de mémoire parmi : mémoire relationnelle, mémoire épisodique, mémoire sémantique, mémoire déclarative, mémoire temporelle, mémoire spatiale, mémoire spatio-temporelle, mémoire de travail, mémoire à court terme, navigation et orientation.

5. Système selon l'une quelconque des revendications 1 à 3,
dans lequel la région sélectionnée du cerveau du participant comprend en outre une ou plusieurs régions parmi : région du cortex périhinal, région du cortex parahippocampique, région du cortex orbito-frontal, région du cortex temporal, région du cortex pariétal, région du cortex occipital, région du cortex frontal, région de l'amygdale et région du noyau caudé.

6. Système selon l'une quelconque des revendications 1 à 4, comprenant en outre un ou plusieurs modules VR ciblant une discrimination, une attention, une perception, des tâches duales et des commutations de tâches.

7. Système selon l'une quelconque des revendications 1 à 6 pour une utilisation dans un procédé mis en œuvre par ordinateur pour fournir un environnement de réalité virtuelle (VR) 3D destiné à fournir un environnement de réalité virtuelle (VR) pour traiter ou prévenir un dysfonctionnement neurologique ou psychiatrique, en particulier Alzheimer, ou des déficiences cognitives dans un vieillissement normal en stimulant une zone cible du cerveau.

FIG. 1

FIG. 2

fMRI activity in Young Adult Spatial Learners

FIG. 3A

fMRI activity in Young Adult Response Learners

FIG. 3B

FIG. 3C  Structural MRI correlates of Strategies in Young Adults

N = 31; t = 3.55 (25.4, -38.5, -4.6)

FIG. 3D  Structural MRI correlates of Strategies in Older Adults

Mean age = 66.6 SD = 6.8
mean MMSE = 29.0 SD = 1.1
mean MoCA = 26.7 SD = 2.1

# FIG. 4

Correlation between strategies and risk of dementia in Older Adults

FIG. 5

| Group | Random # | Rank |
|-------|----------|------|
| A | 0.884 | 3 |
| A | 0.432 | 2 |
| B | 0.207 | 1 |
| B | 0.898 | 4 |

| Group | Random # | Rank |
|-------|----------|------|
| B | 0.207 | 1 |
| A | 0.432 | 2 |
| A | 0.884 | 3 |
| B | 0.898 | 4 |

Group A: SMIP
Group B: Placebo control

**BAAB**

1. Random numbers are generated and ranked in ascending order.

2. Random numbers are sorted in ascending order along with the group they are associated to.

3. The sequence generated is BAAB.

# FIG. 6A

| Strata | Age (yrs) | Sex | Education (yrs) | Group Assignment |
|--------|-----------|-----|-----------------|------------------|
| 1 | 65-69 | M | =11 | BAAB, ABBA, ... |
| 2 | 65-69 | M | >11 | ... |
| 3 | 65-69 | F | =11 | ... |
| 4 | 65-69 | F | >11 | ... |
| 5 | 70-75 | M | =11 | ... |
| 6 | 70-75 | M | >11 | ... |
| 7 | 70-75 | F | =11 | ... |
| 8 | 70-75 | F | >11 | ... |

# FIG. 6B

**Discrimination and Spatial Memory**

*Discrimination*

A1-1roomshapes
A2-bigroomshapes
B1-4rooms
B2-4roomsobjects2
B3-4roomsobjects1
B4-4roomsshapes
C1-9rooms
C2-9roomsobjects

*Discrimination and Spatial Memory*

D1-search1
D2-search2
D3-search3
D4-search4

E1-mallmorning/mallnight
E2-museum
E3-room1
E4-suite
E5-house
E6-house2

**Object Location**

*Basic Objects*

F1-objects2_1
F2-objects2_2
F3-objects4_1
F4-objects4_2
F5-objects6_1
F6-objects6_2
F7-objects8_1
F8-objects8_2
F9-objects10_1
F10-objects10_1
F11-objects10_2
F12-objects12_1
F13-objects12_2

*Basic Shapes*

G1-shapes1_1
G2-shapes1_2
G3-shapes3_1
G4-shapes5_1
G5-shapes5_2
G6-shapes7_1
G7-shapes7_2

**Spatio-temporal Order**

H1-sto1
H2-sto2
H3-sto3
H4-sto4

**Navigation**

I1-navigation1
I2-outside
I3-island
I4-metropolis

# FIG. 7

FIG. 8A

800A

FIG. 8B

800B

FIG. 9

900

FIG. 10A

1000A

FIG. 10B

1000B

FIG. 11

1100A

FIG. 12A

1200A

FIG. 12B

1200B

FIG. 13                    1300

FIG. 14A

1400A

FIG. 14B

1400B

FIG. 15A

1500A

FIG. 15B

1500B

FIG. 15C

1500C

FIG. 15D

1500D

FIG. 15E

1500E

FIG. 15F

1500F

FIG. 15G ⬉ ⌐1500G

FIG. 15H ⬉ ⌐1500H

FIG. 15I ⬉ ⌐1500I

FIG. 15J ⬉ ⌐1500J

FIG. 16A

—1600A

FIG. 16B

—1600B

FIG. 17

1700

FIG. 18

1800

FIG. 19

1900

FIG. 20

2000

FIG. 21

2100

FIG. 22

| Experimental<br>Healthy: Sessions 1 to 4<br>MCI : Sessions 1 – 4 including MRI | Placebo Control<br>Healthy: Sessions 1 to 4<br>MCI : Sessions 1 -4 including MRI |
|---|---|

Transfer Tests
Balanced for order of administration, time of day (AM vs PM) that participants get tested in each group

*Session 1: Neuropsychological tests, 4/8VM, Wayfinding in the Virtual Town*

*Session 1: Neuropsychological tests, 4/8VM, Wayfinding in the Virtual Town*

*Session 2: Neuropsychological tests, self-administered questionnaires*

*Session 2: Neuropsychological tests, self-administered questionnaires*

*Session 3: Mock Scanning Session. Task administered: Go/No-Go*

*Session 3: Mock Scanning Session. Task administered: Go/No-Go*

*Session 4: fMRI scan - task administered: Concurrent Spatial Discrimination Learning Task*

*Session 4: fMRI scan - task administered: Concurrent Spatial Discrimination Learning Task*

Training

16 one-hour Spatial Memory Intervention

16 one-hour Placebo Control

Transfer Tests
Different versions of all virtual navigation tests and Neuropsychological tests are provided, balanced for the version administered before or after training

*Session 1: Neuropsychological tests, 4/8VM, Wayfinding in the Virtual Town*

*Session 1: Neuropsychological tests, 4/8VM, Wayfinding in the Virtual Town*

*Session 2: Neuropsychological tests, self-administered questionnaires*

*Session 2: Neuropsychological tests, self-administered questionnaires*

*Session 3: Mock Scanning Session. Task administered: Go/No-Go*

*Session 3: Mock Scanning Session. Task administered: Go/No-Go*

*Session 4: fMRI scan - task administered: Concurrent Spatial Discrimination Learning Task*

*Session 4: fMRI scan - task administered: Concurrent Spatial Discrimination Learning Task*

# FIG. 23

Session 1 - for Healthy Elderly and MCI
MMSE – Mini-Mental Status Examination
4/8VM - spatial vs. response strategies (version 1 or 2)
MoCA – Montreal Cognitive Assessment
Wayfinding - Virtual Town (version 1 or 2)
GDS- Geriatric Depression Scale
Stroop

Session 2 - for Healthy Elderly and MCI
Toni-3 non verbal IQ (version A or B)
Trails
BIS - Barratt Impulsiveness Scale
FSAQ - Functional Spatial Abilities Questionnaire
DST - Digit Symbol Test
R/T – Rey Osterrieth Complex Figure or Taylor Complex Figure
RAVLT - Rey Auditory Verbal Learning Task
SEQ – Self-Esteem Questionnaire
PSS - Perceived Stress Scale
QLQ - Quality of Life Questionnaire

Session 3 - Go/No-Go (version 1 or 2) - for Healthy Elderly and MCI
    *The Go/No-Go is administered during a Mock MRI Scanning Session for Healthy Elderly
    and MCI in order to practice for fMRI*

Session 4 - Concurrent Spatial Discrimination Learning Task (CSDLT) (version 1 or 2) - for
    Healthy Elderly and MCI
    *The CSDLT is administered during an fMRI Scanning Session for Healthy Elderly and MCI*

# FIG. 24

- **MoCA/MMSE**
  - − Test sensitive to cognitive impairment.

- **Geriatric Depression Scale (GDS)**
  - − Assessment of mood tailored to older adults.

- **Test of Nonverbal Intelligence (TONI-3)**
  - − Non-verbal IQ test.

- **Quality of Life (QOL)**
  - − Multi-domain assessment (mood, stress, social interactions, marital and professional satisfaction, leisure opportunities, alcohol/drug use.).

- **Self-Esteem Questionnaire (SEQ)**
  - − Measure of self-esteem.

- **Cohen Perceived Stress Scale (PSS)**
  - − Subjective measure of stress perception.

- **Barratt's Impulsiveness Scale (BIS)**
  - − Measure of Impulsivity across 3 domains: attentive, motor and planning.

- **Functional Spatial Abilities Questionnaire (FSAQ)**
  - − Incidences of wayfinding difficulties (getting lost, misplacing items).

- **Stroop/Trails A & B**
  - − Measure of reaction times, mental flexibility, attention.

# FIG. 25

FIG. 26A

| List B (Interference) | | List A (After Interference) | | List A (30-min Delayed Recall) | |
|---|---|---|---|---|---|
| desk | ____ | drum | ____ | drum | ____ |
| ranger | ____ | curtain | ____ | curtain | ____ |
| bird | ____ | bell | ____ | bell | ____ |
| shoe | ____ | coffee | ____ | coffee | ____ |
| stove | ____ | school | ____ | school | ____ |
| mountain | ____ | parent | ____ | parent | ____ |
| glasses | ____ | moon | ____ | moon | ____ |
| towel | ____ | garden | ____ | garden | ____ |
| cloud | ____ | hat | ____ | hat | ____ |
| boat | ____ | farmer | ____ | farmer | ____ |
| lamb | ____ | nose | ____ | nose | ____ |
| gun | ____ | turkey | ____ | turkey | ____ |
| pencil | ____ | color | ____ | color | ____ |
| church | ____ | house | ____ | house | ____ |
| fish | ____ | river | ____ | river | ____ |
| Total | ____ | Total | ____ | Total | ____ |
| Time | ____ | Time | ____ | Time | ____ |

FIG. 26B

FIG. 27

FIG. 28A

FIG. 28B

FIG. 28C

FIG. 29A

FIG. 29B

FIG. 30

*PI: Percent Improvements:*

$$PI = AI/Average\ (et1, et2, ct1, ct2)*100$$

*AI:* Absolute Improvements:

$$AI = (Average\ (et1n - et2n)) - (Average\ (ct1n - ct2n))$$

*et1*: Experimental Transfer 1
*et2*: Experimental Transfer 2
*ct1*: Control Transfer 1
*ct2*: Control Transfer 2
*n1*: subject 1, *n2*: subject 2...

The Absolute Improvement scores do not allow for comparisons between tasks, as they represented values which differed in scale and units of measure. For example, the MoCA was measured by adding scores, whereas the Wayfinding task was measured as path lengths and latencies. As such, we pooled the averages of each task for both groups and both time points and divided the AI by this average pool to obtain a Percent Improvement (PI) representing a measure of improvement comparable across tasks regardless of units of measure or scale they reflected.

# FIG. 31

**FIG. 32A**

3200A

**FIG. 32B**

3200B

FIG. 33A

—3300A

FIG. 33B

—3300B

A) Post-minus Pre-Memory Training functional MRI in Healthy

3400A

1.5    3.75    6.0

N = 7; t = 3.6, p < 0.006

(-27, -11.7, -28.1)

## FIG. 34A

B) Post-minus Pre-Control functional MRI in Healthy

3400B

1.5    3.75    6.0

N = 7; t = -0.13, p > 0.05

(-27.0, -11.7, -28.1)

## FIG. 34B

A)   Post-minus Pre-Memory Training structural MRI in Healthy

3500A

0.5        2.25        4.0

N = 7; t = 1.64

(-29.0, -21.8, -19.9)

FIG. 35A

B)   Post-minus Pre-Control structural MRI in Healthy

3500B

0.5        2.25        4.0

N = 7; t = -0.44

(-29.0, -21.8, -19.9)

FIG. 35B

## Discrimination

Participant 1  ⁼Participant 2  ▪Participant 3

3600A

# FIG. 36A

## Discrimination & Spatial Memory

Participant 1  ⁼Participant 2  ▪Participant 3

Last trial

3600B

# FIG. 36B

## Object Location

Participant 1  ⁼Participant 2  ▪Participant 3

Number of Objects

3600C

# FIG. 36C

FIG. 37A

FIG. 37B

FIG. 38A

FIG. 38B

FIG. 38C

FIG. 39A

FIG. 39B

4000A

**Counting Forwards and Backwards from 1000**

■ Subtract 3 from 1000    □ Add 3 from 1000

Percent Correct

100
80
60
40
20
0

1    2    3    4    5    6    7    8

**Stop Number**

# FIG. 40A

**Counting Forwards and Backwards from 100**

Percent Correct

100
80
60
40
20
0

Alternate    3    2

**Number of Lanterns on the same side**

4000B

# FIG. 40B

**Memorizing numbers of colored cards encountered along a path**

Percent Correct

100
80
60
40
20
0

4    5    6    7

**Number of Panels to Memorize**

4000C

# FIG. 40C

## Placebo Control for the Spatial Memory Intervention Program
### Educational documentary

DVD          Quiz          Feed-back

1. How long each year are penguins in the dark in Antarctica?

☐ 4 months
☐ 4 days
☐ all year long
☐ 4 hours

2. How many polar bear cubs were there?

☐ none
☐ 1
☐ 2
☐ 5

FIG. 41          4100

Pre-Memory Training structural MRI in MCI patients

FIG. 42

4200

## Pre-SMIP MCI

2 4 6

N = 4; *t* = -0.29, *p* > 0.05

(-27, -11.7, -28.1)

4300A

FIG. 43A

## Pre-SMIP Healthy

2 4 6

N = 9; *t* = -2.09, *p* > 0.05

(-27, -11.7, -28.1)

4300B

FGI. 43B

Post-minus Pre-Memory Training MCI

FIG. 44

FIG. 45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOHBOT et al.** Gray matter differences correlate with spontaneous strategies in a human virtual navigation task. *THE JOURNAL OF NEUROSCIENCE,* 19 September 2007, vol. 27 (38), 10078-10083